(19) Europäisches Patentamt
European Patent Office
Office européen des brevets

(11) EP 3 169 775 B1

(12) # EUROPÄISCHE PATENTSCHRIFT

(45) Veröffentlichungstag und Bekanntmachung des Hinweises auf die Patenterteilung:
**30.07.2025 Patentblatt 2025/31**

(21) Anmeldenummer: **15715264.6**

(22) Anmeldetag: **10.04.2015**

(51) Internationale Patentklassifikation (IPC):
***C12N 9/10*** (2006.01) ***A61K 31/702*** (2006.01)

(52) Gemeinsame Patentklassifikation (CPC):
**C12P 19/26; C12N 9/1051; C12N 9/1241; C12N 9/90; C12N 15/52; C12P 19/18; C12Y 204/01; C12Y 204/01022; C12Y 204/01062; C12Y 204/01146; C12Y 207/01052; C12Y 207/0703; C12Y 207/07064; C12Y 501/03002**

(86) Internationale Anmeldenummer:
**PCT/EP2015/057805**

(87) Internationale Veröffentlichungsnummer:
**WO 2016/008602 (21.01.2016 Gazette 2016/03)**

(54) **BIOTECHNOLOGISCHE HERSTELLUNG VON LNT, LNNT UND DEREN FUCOSYLIERTEN DERIVATEN**

BIOTECHNOLOGICAL PRODUCTION OF LNT, LNNT AND THEIR FUCOSYLATED DERIVATIVES

FABRICATION BIOTECHNOLOGIQUE DE LNT, LNNT ET LEURS DÉRIVÉS FUCOSYLÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.07.2014 EP 14176958**
**18.12.2014 EP 14198960**

(43) Veröffentlichungstag der Anmeldung:
**24.05.2017 Patentblatt 2017/21**

(73) Patentinhaber: **BASF SE**
**67056 Ludwigshafen am Rhein (DE)**

(72) Erfinder:
- **BAUMGÄRTNER, Florian**
  **70174 Stuttgart (DE)**
- **SPRENGER, Georg, A.**
  **71522 Backnang (DE)**
- **ALBERMANN, Christoph**
  **42369 Wuppertal (DE)**

(74) Vertreter: **BASF IP Association**
**BASF SE**
**GBI - Z078**
**67056 Ludwigshafen (DE)**

(56) Entgegenhaltungen:
**EP-A1- 1 316 608**
**WO-A1-01/04341**
**WO-A1-2013/182206**
**WO-A2-2012/112777**
**US-A1- 2003 013 175**

- **CHRISTOPH ALBERMANN ET AL: "A simple and reliable method to conduct and monitor expression cassette integration into the Escherichia coli chromosome", BIOTECHNOLOGY JOURNAL, vol. 5, no. 1, 1 January 2010 (2010-01-01), DE, pages 32 - 38, XP055687266, ISSN: 1860-6768, DOI: 10.1002/biot.200900193**
- **PRIEM BERNARD ET AL: "A new fermentation process allows large-scale production of human milk oligosaccharides by metabolically engineered bacteria", GLYCOBIOLOGY, OXFORD UNIVERSITY PRESS, US, vol. 12, no. 4, 1 April 2002 (2002-04-01), pages 235 - 240, XP002380632, ISSN: 0959-6658, DOI: 10.1093/GLYCOB/12.4.235**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 169 775 B1

## Beschreibung

Die vorliegende Erfindung betrifft gentechnisch veränderte Mikroorganismen zur in vivo-Synthese von Lacto-N-tetraose (LNT) bzw. Lacto-N-neotetraose (LNnT) und deren fucosylierten Derivaten in einem Mikroorganismus, sowie Verwendungen solcher Mikroorganismen in Verfahren zur Herstellung von Lacto-N-tetraose bzw. Lacto-N-neotetraose und deren fucosylierten Derivaten.

Menschlicher Muttermilch wird eine wichtige Rolle bei der gesunden Kindesentwicklung beigegemessen. Die darin vorkommenden Oligosaccharide (human milk oligosacchraides (HMO)) sind eine der festen Hauptkomponenten der Muttermilch und haben eine Kernstruktur, die eine Lactoseeinheit am reduzierenden Ende aufweist und mit N-Acetyllactosamineinheiten verzweigt oder kettenförmig fortgesetzt wird. Die strukturelle Variabilität wird zudem durch Fucosyl- oder Sialyl-Modifikationen an den Endpositionen erweitert.

Bei Lacto-N-tetraose (LNT) handelt es sich um ein Tetrasaccharid der chemischen Formel N-[(2S,3R,4R,5S,6R)-2-{[(2R,3S,4S,5R,6S)-3,5-Dihydroxy-2-(hydroxymethyl)-6-{[(2R,3S,4R,5R)-4,5,6-trihydroxy-2-(hydroxymethyl)oxan-3-yl]oxy}oxan-4-yl]oxy}-5-hydroxy-6-(hydroxymethyl)-4-{[(2R,3R,4S,5R,6R)-3,4,5-trihydroxy-6-(hydroxymethyl)oxan-2-yl]oxy}oxan-3-yl]acetamid mit folgender Struktur:

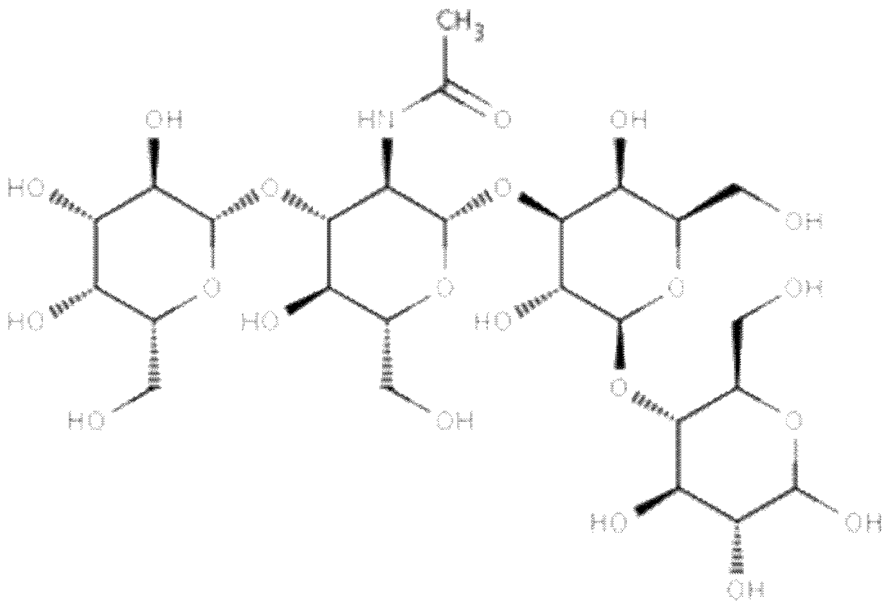

Lacto-N-neotetraose (LNnT) hat die chemische Formel N-[(2S,3R,4R,5S,6R)-2-{[(2R,3S,4S,5R,6S)-3,5-Dihydroxy-2-(hydroxymethyl)-6-{[(2R,3R,4R,5R)-1,2,4,5-tetrahydroxy-6-oxohexan-3-yl]oxy}oxan-4-yl]oxy}-4-hydroxy-6-(hydroxymethyl)-5-{[(2S,3R,4S,5R,6R)-3,4,5-trihydroxy-6-(hydroxymethyl)oxan-2-yl]oxy}oxan-3-yl]acetamid bzw. folgende Struktur:

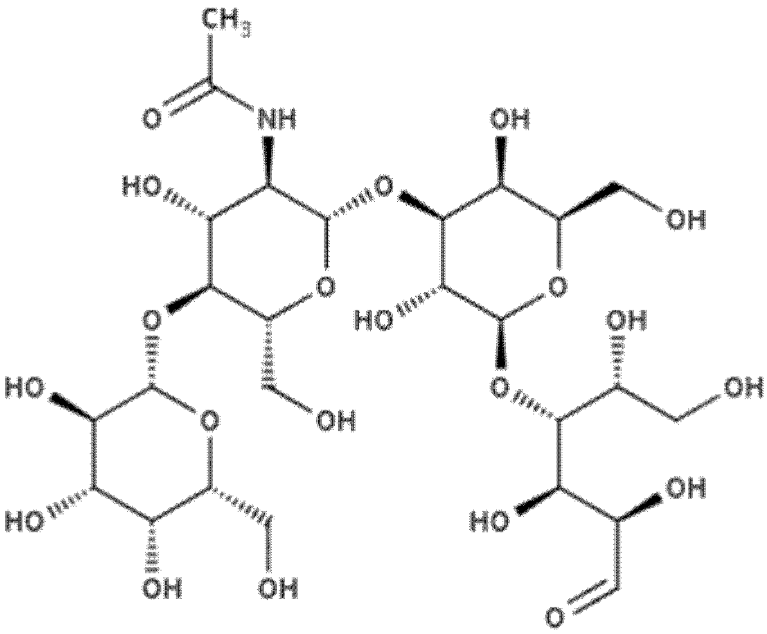

Im Hinblick auf die gesundheits- und entwicklungsfördernde Wirkung ist die biologische Funktion der HMOs Gegenstand zahlreicher Studien, erfordert jedoch die technische Reingewinnung der Verbindungen in ausreichenden Mengen. Die ziemlich aufwendige Extraktion aus Muttermilch ist derzeit die am meisten angewandte Methode. Biotechnologische Produktionsverfahren wurden zwar für HMOs beschrieben (siehe Han et al., Biotechnol. Adv. 2012, 30, 1268-1278), jedoch ist beispielsweise Lacto-N-tetraose als eines der häufigsten HMOs derzeit nicht zu einem angemessenen Preis für die Forschung erhältlich. Sowohl chemische als auch enzymatische Synthesen für LNT sind aus der Literatur bekannt (siehe Aly et al., Carbohydr. Res. 1999, 316, 121-132; Murata et al., Glycoconj. J. 1999, 16, 189-195). Da jedoch die chemische Synthese mehrere Schritte der Schützung und Entschützung reaktiver Gruppen erfordert und die enzymatische Synthese von unvorteilhaften Gleichgewichtsproduktverteilungen und Regioselektivitäten betroffen ist, liefern diese Methoden keine zufriedenstellenden Ergebnisse.

Primäre Aufgabe der vorliegenden Erfindung war es daher, ein System, vorzugsweise Mikroorganismen, anzugeben, das bzw. die dazu in der Lage ist bzw. sind, LNT bzw. LNnT und deren fucosylierte Derivate in hoher Ausbeute zu produzieren.

Eine weitere Aufgabe der vorliegenden Erfindung war die Bereitstellung eines entsprechenden Verfahrens, das die effiziente und preiswerte biotechnologische Produktion von LNT und LNnT bzw. deren fucosylierten Derivaten ermöglicht.

Die primäre Aufgabe wird erfindungsgemäß gelöst durch einen gentechnisch veränderten Mikroorganismus zur in vivo-Synthese von Lacto-N-tetraose oder Lacto-N-neotetraose in einem Mikroorganismus, wobei der Mikroorganismus

(i) ein erstes Transgen, das für β 1,3-N-Acetylglucosaminyltransferase codiert,
und

(ii) ein zweites Transgen, das für β 1,3-Galactosyltransferase (im Falle der Synthese von Lacto-N-tetraose) oder für β 1,4-Galactosyltransferase (im Falle der Synthese von Lacto-N-neotetraose) codiert,

enthält, und wobei der Mikroorganismus zudem so gentechnisch verändert ist, dass die Expression von LacZ und LacA unterdrückt wird, wobei das (i) erste Transgen in den LacZYA-Lokus integriert ist und der Mikroorganismus ein weiteres Transgen enthält, das für LacY codiert.

Unter einem gentechnisch veränderten Mikroorganismus ist in diesem Zusammenhang ein Mikroorganismus zu verstehen, in dem gezielt mit biotechnologischen Methoden einzelne Gene ausgeschaltet und/oder arteigene oder artfremde Gene eingebaut wurden (Transgene). Bei einem Transgen im Sinne der vorliegenden Erfindung kann es sich um ein eingeschleustes Gen aus einem anderen Organismus oder auch um ein natürlicherweise in dem betreffenden Mikroorganismus vorkommendes Gen handeln, das gentechnisch an anderer Stelle im Genom integriert worden ist und daher beispielsweise unter einem anderen als dem natürlichen Promoter exprimiert wird.

Im Rahmen der vorliegenden Erfindung wurde überraschenderweise herausgefunden, dass standardmäßig gentechnisch eingesetzte Mikroorganismen, die β 1,3-*N*-Acetylglucosaminyltransferase und eine β 1,3-Galactosyltransferase bzw. β 1,4-Galactosyltransferase transgen exprimieren, erfolgreich zur Synthese von LNT bzw. LNnT eingesetzt

werden können. Dabei können beispielsweise Leloir Glycosyltransferasen (LgtA bzw. LgtB) verwendet werden, die Lactose als Substrat für die Glycosylierung zunächst zu Lacto-N-Triose II (LNT II) als Zwischenprodukt umsetzen und dann in einem von Nucleotid-aktivierten Zuckern abhängigen Schritt zu LNT elongieren (siehe Fig. 1 sowie Frey et al. FASEB J. 1996, 10, 461- 70). Als geeignete Transgene haben sich im Rahmen der Erfindung beispielsweise das für die β 1,3-*N*-Acetylglucosaminyltransferase codierende lgtA-Gen aus *Neisseria meningitides* und das für die β 1,3-Galactosyltransferase codierende wbgO-Gen aus *E. coli* erwiesen. Die Donor-Substrate der rekombinanten Glycosyltransferasen LgtA (UDP-N-Acetylglucosamin) und WbgO (UDP-Galactose) sind Zwischenprodukte des *E. coli* K12-Metabolismus und werden während des Wachstums ständig synthetisiert (siehe Raetz et al., Annu. Rev. Biochem., 2002, 71, 635-700). UDP-N-Acetylglucosamin ist ein Vorläufer der Peptidoglycan-, Lipopolysaccharid- und enterobaketeriellen gemeinsamen Antigen-Biosynthese (siehe Neidhardt et al., Cellular and Molecular Biology, second edition 1996). Es wird aus Fructose-6-phosphat durch die Biosynthese-Enzyme GlmS, GlmM, and GlmU hergestellt (siehe Barreteau et al., FEMS Microbiol. Rev., 2008, 32, 168-207). UDP-Galactose ist ein Vorläufer-Substrat der Lipopolysaccharid- und der Colanicsäure-Biosynthese in *E. coli* und wird aus Glucose-6-phosphat in drei enzymatischen Schritten, katalysiert durch Pgm, GalU und GalE, gebildet (siehe Frey, FASEB J., 1996, 10, 461-70). Für das Zellwachstum und die intrazelluläre Bereitstellung der Nucleotid-aktivierten Zucker können vorteilhafterweise preiswerte Substrate wie Glycerin oder Glucose eingesetzt werden.

Gemäß der vorliegenden Erfindung ist der Mikroorganismus gentechnisch so verändert, dass die Expression von LacZ und LacA unterdrückt wird. Dabei ist das (i) erste Transgen in den LacZYA-Lokus integriert und der Mikroorganismus enthält ein weiteres Transgen, das für LacY codiert.

Um den Metabolismus und mögliche Acetylierung von Lactose durch LacZ und LacA zu verhindern, wird die Expression dieser Gene in einem erfindungsgemäßen Mikroorganismus unterdrückt. Erfindungsgemäß erfolgt dies über die Integration des ersten Transgens (i) in den LacZYA-Lokus. Damit jedoch die Lactose-Aufnahme des Mikroorganismus weiterhin gewährleistet bleibt, wird LacY an anderer Stelle im Genom, beispielsweise unter einem anderen Promoter, vorzugsweise einem $P_{tac}$-promoter, transgen exprimiert. Besonders bevorzugt wird lacY in den fuclK-Lokus integriert, der für Gene des Fucose-Metabolismus codiert.

Um eine möglichst hohe Ausbeute an LNT bzw. LNnT zu gewährleisten ist es von Vorteil, reichlich Nucleotid-aktivierte Zucker, insbesondere UDP-Galactose, intrazellulär zur Verfügung zu stellen, damit die Umsetzung von LNT II zu LNT effizient ablaufen kann.

Demgemäß enthält in einer weiteren bevorzugten Ausführungsform der vorliegenden Erfindung der Mikroorganismus ein weiteres Transgen, das für eine UDP-Zucker-Pyrophosphorylase (USP) codiert.

Eine solche USP wird beispielsweise durch den offenen Leserahmen LmjF17.1160 in *Leishmania major* codiert (siehe Damerow et al., J.Biol. Chem. 2010, 285, 878-887). Die USP katalysiert die Erstellung von UDP-Galactose unter Verwendung von Galactose-1-Phosphat. Vorteilhafterweise kann durch diese Umsetzung auch einer unter Umständen zelltoxischen Akkumulation von Galactose-1-Phosphat vorgebeugt werden.

In einer weiteren bevorzugten Ausführungsform der vorliegenden Erfindung ist der Mikroorganismus zudem gentechnisch so verändert, dass die Expression von UDP-Glucose-4-Epimerase unterdrückt wird.

Eine solche Unterdrückung kann beispielsweise durch Deletion des galE-Gens erreicht werden, welches bevorzugt durch einen T5-Promoter ersetzt wird, so dass die folgenden Gene des Operons weiterhin exprimiert werden. Vorteilhafterweise wird somit ebenfalls die intrazelluläre UDP-Galactose-Konzentration erhöht. Besonders bevorzugt ist eine Kombination dieser Ausführungsform mit dem Mikroorganismus enthaltend ein Transgen, das für eine UDP-Zucker-Pyrophosphorylase (USP) codiert (wie oben beschrieben).

Erfindungsgemäß besonders bevorzugt ist weiterhin ein Mikroorganismus nach einer der oben beschrieben Ausführungsformen, bei dem ein oder beide bzw. ein, mehrere oder sämtliche Transgene chromosomal integriert ist bzw. sind.

Die Verwendung eines Plasmid-freien Stammes ist besonders vorteilhaft, da zur Erhaltung der Produktivität kein Selektionsdruck (Antibiotika-Resistenzen) notwendig ist. Zudem ist die Verwendung von Antibiotika in Nahrungsmittelbezogenen oder pharmazeutisch anzuwendenden Produktionen nicht wünschenswert.

Gemäß einer weiteren bevorzugten Ausführungsform des erfindungsgemäßen Mikroorganismus enthält dieser ein weiteres Transgen, das für ein bifunktionales Enzym mit L-Fucose-Kinase-Aktivität und L-Fucose-1-Phosphat-Guanylyltransferase-Aktivität codiert sowie mindestens ein Transgen, welches für ein Enzym codiert, welches zur α (alpha)1,2-Fucosylierung, α (alpha)1,3-Fucosylierung oder α (alpha)1,4-Fucosylierung befähigt ist.

Ein solcher Mikroorganismus ist in der Lage, als Folgereaktion der Synthese von LNT bzw. LNnT die fucosylierten Derivate dieser beiden Verbindungen herzustellen und somit die Anwendungsmöglichkeiten des erfindungsgemäßen Mikroorganismus im Hinblick auf die strukturelle Variabilität der natürlich vorkommenden HMOs zu erweitern (siehe Fig. 2). Beispielsweise kann FKP als ein bifunktionales Enzym mit L-Fucose-Kinase-Aktivität und L-Fucose-1-Phosphat-Guanylyltransferase-Aktivität eingesetzt werden. Für die Fucosylierung eignet sich beispielsweise die Expression der Enzyme, die durch die Gene futC (α 1,2-Fucosylierung), fucT14 (α 1,4-Fucosylierung) oder futA (α 1,3-Fucosylierung) codiert werden.

In einer bevorzugten Ausführungsform des erfindungsgemäßen Mikroorganismus (wie oben beschrieben) ist das Transgen, das für das bifunktionale Enzym mit L-Fucose-Kinase-Aktivität und L-Fucose-1-Phosphat-Guanylyltransferase-Aktivität codiert, chromosomal integriert und das mindestens eine Transgen, welches für ein Enzym codiert, welches zur $\alpha$ 1,2-Fucosylierung, $\alpha$ 1,3-Fucosylierung oder $\alpha$ 1,4-Fucosylierung befähigt ist, wird auf einem Plasmidvektor exprimiert.

In einer besonders bevorzugten Ausführungsform des erfindungsgemäßen Mikroorganismus (wie oben beschrieben) sind sowohl das Transgen, das für das bifunktionale Enzym mit L-Fucose-Kinase-Aktivität und L-Fucose-1-Phosphat-Guanylyltransferase-Aktivität codiert, als auch das mindestens eine Transgen, welches für ein Enzym codiert, welches zur $\alpha$ 1,2-Fucosylierung, $\alpha$ 1,3-Fucosylierung oder $\alpha$ 1,4-Fucosylierung befähigt ist, chromosomal integriert.

Ein weiterer Aspekt der vorliegenden Erfindung betrifft die Verwendung eines gentechnisch veränderten Mikroorganismus wie hierin beschrieben, vorzugsweise wie hierin gemäß einer der oben beschriebenen Ausführungsformen als bevorzugt beschrieben, zur in vivo-Synthese von Lacto-N-tetraose oder Lacto-N-neotetraose oder eines fucosylierten Derivats von Lacto-N-tetraose oder Lacto-N-neotetraose in einem Mikroorganismus.

Die Verwendung eines solchen gentechnisch veränderten Mikroorganismus ermöglicht die effiziente und preiswerte Herstellung von Lacto-N-tetraose oder Lacto-N-neotetraose oder eines fucosylierten Derivats von Lacto-N-tetraose oder Lacto-N-neotetraose in einem Maßstab, der an die beabsichtigte Verwendung angepasst werden kann.

Gemäß einem weiteren Aspekt betrifft die vorliegende Erfindung ein Verfahren zur Herstellung von Lacto-N-tetraose oder Lacto-N-neotetraose oder eines fucosylierten Derivats von Lacto-N-tetraose oder Lacto-N-neotetraose, umfassend die folgenden Schritte:

(a) Bereitstellen eines gentechnisch veränderten Mikroorganismus wie oben beschrieben, vorzugsweise wie oben als bevorzugt beschrieben,

(b) Kultivieren des gentechnisch veränderten Mikroorganismus unter Bedingungen, die die Synthese von Lacto-N-tetraose bzw. Lacto-N-neotetraose erlauben,

(c) optional Zugeben von Fucose,

(d) optional isolieren der synthetisierten Lacto-N-tetraose bzw. Lacto-N-neotetraose bzw. des fucosylierten Derivats von Lacto-N-tetraose oder Lacto-N-neotetraose.

In dem erfindungsgemäßen Verfahren wird zunächst ein gentechnisch veränderter Mikroorganismus wie oben beschrieben bereitgestellt und beispielweise in einem Schüttelkolben unter Bedingungen, die die Synthese von Lacto-N-tetraose bzw. Lacto-N-neotetraose erlauben, kultiviert. Das Zellwachstum hängt dabei in erster Linie von dem eingesetzten Mikroorganismus ab. Bevorzugt handelt es sich bei dem eingesetzten Mikroorganismus um einen standardmäßig verwendeten Mikroorganismus für biotechnologische Anwendungen, der für eine maximale Produktivität optimiert ist. Vorteilhafterweise können neben der notwendigen Lactose preiswerte (weitere) Kohlenstoffquellen, beispielsweise ausgewählt aus der Gruppe bestehend aus Glucose, Glyzerin, Galactose und beliebige Mischungen derselben, verwendet werden. Um die Synthese von LNT bzw. LNnT zu ermöglichen, muss Lactose als Substrat vorhanden sein und die Expression der Transgene (i) und (ii) (wie oben beschrieben) gegebenenfalls abhängig von dem Promoter unter dem sie exprimiert werden, induziert werden. Um die Fucosylierung der Produkte zu gewährleisten, muss zudem Fucose zugegeben werden. Die Fucose-Zugabe erfolgt vorzugsweise erst nach der Induktion der Gene für die LNT- bzw. LNnT-Synthese, idealerweise so, dass ausreichend entsprechendes Substrat vorhanden ist und nicht lediglich Lactose fucosyliert wird. Alternativ kann die Fucose auch bereits zu Beginn von Schritt (b) vorhanden sein und die Expression unter einen anderen als den die Expression der Gene für die LNT- bzw. LNnT-Synthese regulierenden Promoter gestellt werden. Die Induktion der Fucosyltransferasegene erfolgt dann zur gewünschten Zeit durch Zugabe des entsprechenden Induktionsmittels. In einer bevorzugten Ausgestaltung erfolgen in diesem Falle die Expression der Gene für die LNT- bzw. LNnT-Synthese unter einem IPTG-induzierbaren Promoter und die Expression der Fucosyltransferasegene unter einem Rhamnose-induzierbaren Promoter.

Anschließend werden optional die hergestellten Produkte isoliert. Dazu werden die Zellen beispielsweise mittels Zentrifugation gesammelt, in Wasser resuspendiert und lysiert. Aus dem Überstand können dann die hergestellten Zucker unter Verwendung von Standardmethoden aufgereinigt werden.

In einer bevorzugten Ausführungsform des erfindungsgemäßen Verfahrens wird bzw. werden in Schritt (b)

- Galactose als Kohlenstoffquelle für den Mikroorganismus verwendet
  oder

- Glyzerin und Galactose als Kohlenstoffquelle für den Mikroorganismus verwendet.

Im Rahmen der vorliegenden Erfindung wurde herausgefunden, dass sich die Ausbeute von LNT im Verhältnis zu dem Zwischenprodukt LNT II über die zur Verfügung gestellten Kohlenstoffquellen steuern lässt (siehe Fig. 3). Besonders hohe Ausbeuten ergeben sich demnach beispielsweise dann, wenn Galactose als primäre Kohlenstoffquelle vorliegt. Bevorzugt beträgt der (Gew.-)Anteil an Galactose, bezogen auf das Gesamtgewicht an der für die Synthese notwendigen Lactose und gegebenenfalls anderen vorhandenen Kohlenstoffquellen wie z.B. Glyzerin oder Glucose, wenigstens 50%, vorzugweise 70%, besonders bevorzugt wenigstens 90%. Ebenfalls besonders hohe Ausbeuten werden erzielt, wenn Glyzerin als primäre Kohlenstoffquelle verwendet wird und Galactose bei Beginn der Induktion der Gene für die LNT- bzw. LNnT-Synthese zugegeben wird. Wiederum beträgt der (Gew.-)Anteil des Glyzerin, bezogen auf das Gesamtgewicht an der für die Synthese notwendigen Lactose und gegebenenfalls anderen vorhandenen Kohlenstoffquellen wie z.B. Glucose, wenigstens 50%, vorzugweise wenigstens 70%, besonders bevorzugt wenigstens 90%.

Bevorzugt ist weiterhin ein erfindungsgemäßes Verfahren (wie oben beschrieben), in dem in Schritt (b) kontinuierlich oder schrittweise eine oder mehrere Kohlenstoffquelle(n), vorzugsweise ausgewählt aus der Gruppe bestehend aus Lactose, Glucose, Glyzerin, Galactose und beliebige Mischungen derselben, bevorzugt wenigstens Lactose, besonders bevorzugt Lactose und Galactose oder Lactose, Galactose und Glyzerin, zugegeben werden.

Vorteilhafterweise können durch kontinuierliche oder schrittweise Zugabe der jeweiligen Kohlenstoffquelle(n), wenn diese vollständig bzw. zu einem bestimmten Teil verbraucht wurden, gegebenenfalls zelltoxische Akkumulationen oder unerwünschte Inhibitionen vermieden werden.

Bevorzugt ist der gentechnisch veränderte Mikroorganismus (wie oben beschrieben) oder der gemäß einer hierin beschriebenen Verwendung oder der in einem hierin beschriebenen Verfahren erfindungsgemäß einzusetzende Mikroorganismus ausgewählt aus der Gruppe bestehend aus Bakterien, Pilzen und Pflanzen, bevorzugt Mikroorganismen der Gattung *Corynebacterium,* insbesondere *Corynebacterium glutamicum, Bevibacterium,* insbesondere *Bevibacterium flavum, Bacillus, Saccharomyces* und *Escherichia,* insbesondere *E. coli.*

Die Verwendung von standardmäßig gentechnisch eingesetzten Mikroorganismen ist für die Ausführung der vorliegenden Erfindung besonders vorteilhaft, da diese für eine hohe Produktivität optimiert wurden und gentechnische Verfahren zur Einführung von Transgenen und die Induktion derselben bekannt sind.

Im Rahmen der vorliegenden Erfindung wurde demonstriert, dass das erfindungsgemäße Verfahren in Form eines Zulauf-Batch-Prozesses auch im Liter-Maßstab effizient durchgeführt werden kann (siehe Beispiel 3). Bevorzugt ist daher ein Verfahren wie oben beschrieben, wobei das Verfahren als Zulauf-Batch-Prozess mit einem Batch-Volumen im Bereich von 2 bis 30 L, bevorzugt von 3 bis 20 L, besonders bevorzugt von 5 bis 15 L durchgeführt wird.

Im Folgenden wird die Erfindung exemplarisch anhand von Figuren und Beispielen näher erläutert.

Kurzbeschreibung der Figuren:

**Figur 1:** Schema des intrazellulären Umsatzes von Lactose zu Lacto-N-Tetraose.

**Figur 2.** Schema der intrazellulären Synthese fucosylierter HMOs mit LNT als Kernstruktur. FucT steht dabei für eine Fucosyltransferase und LNFX für die daraus entstehenden Produkte.

**Figur 3:** Anteil des jeweiligen Oligosaccharids in Schüttelkolbenkulturen im Kulturüberstand an der Gesamtmenge des Oligosaccharids 24 Stunden nach Induktion in Abhängigkeit der Kohlenstoffquellen. Induktion mit 0,5 mM IPTG und Zugabe von 2 g $l^{-1}$ Lactose und 2 g $l^{-1}$ der jeweils an zweiter Stelle aufgeführten Kohlenstoffquelle und Inkubation bei 30°C und 90 rpm.

**Figur 4:** LNT-Konzentrationen in Schüttelkolbenkulturen 24 Stunden nach Induktion in Abhängigkeit der Kohlenstoffquellen. Induktion mit 0,5 mM IPTG und Zugabe von 2 g $l^{-1}$ Lactose und 2 g $l^{-1}$ der jeweils an zweiter Stelle aufgeführten Kohlenstoffquelle und Inkubation bei 30°C und 90 rpm.

**Figur 5:** LNT II Konzentrationen in Schüttelkolbenkulturen 24 Stunden nach Induktion in Abhängigkeit der Kohlenstoffquellen. Induktion mit 0,5 mM IPTG und Zugabe von 2 g $l^{-1}$ Lactose und 2 g $l^{-1}$ der jeweils an zweiter Stelle aufgeführten Kohlenstoffquelle und Inkubation bei 30°C und 90 rpm.

**Figur 6:** Struktur von LNF I (LNT mit einem $\alpha$ 1,2-verknüpften Fucosylrest am Galactosylrest am nicht-reduzierenden Ende).

**Figur 7:** Struktur von LND II (LNT mit einem $\alpha$ 1,4-verknüpften Fucosylrest am N-Acetylglucosaminylrest und einem $\alpha$

1,3-verknüpften Fucosylrest am Glycosylrest am reduzierenden Ende).

**Figur 8:** Vergleich des Lactose-Verbauchs und der Produktbildung in den Schüttelflaschen-Experimenten auf verschiedenen Kohlenstoffquellen 24 Stunden nach der Induktion. **a)** Konzentration von Lactose (weiß), LNT II (grau) und LNT (schwarz). **b)** Produktausbeuten pro Biomasse. **c)** Anteil der Produkte im Kulturüberstand in %.

**Figur 9:** Intrazelluläre Konzentration der UDP-Zucker wärend des exponentiellen Wachstums auf verschiedenen Kohlenstoffquellen: UDP-Glucose (grau), UDP-Galactose (schwarz) UPD-Acetylglucosamin (weiß). Die Werte sind als Mittel und SE für ≥ 2 unabhängige Experimente angegeben.

**Figur 10:** LNT-Zulauf-Batch-Produktion. Vertikale, gestrichelte Linien (12.6 Stunden) zeigen die IPTG-Zugabe für die Induktion der Protein-Expression und eine erste Lactose-Zugabe an. Vertikale, gepunktete Linien (20.5 Stunden) zeigen das ende der Batch-Phase und den Start der Galactose- und Stickstoff-Zugabe an. **a)** Profil der gesamten dem System zugegebenen Kohlenstoffquelle: Galactose (durchgezogene Linie), Stickstoffquelle: Ammoniumphosphat (gepunktete Linie) und Lactose (gestrichelte Linie); **b)** Zell-Trockengewicht Konzentration (CDW); **c)** Konzentration von LNT II (offene Dreiecke) und LNT (gefüllte Kreise).

**Figur 11:** Struktur der fucosylierten Lacto-N-triose II.

**Figur 12:** Struktur der difucosylierten Lacto-N-Pentaose.

Beispiel 1: Herstellung eines erfindungsgemäßen gentechnisch veränderten Mikroorganismus

Der *E. coli* K-12 Stamm LJ110 wurde als Ausgangsstamm für die Herstellung verwendet. Dieser Plasmid-freie Stamm wurde durch knock-out von Gen-Loci für den Zuckerabbau in den entsprechenden Expressionskassetten mittels homologer Rekombination modifiziert. Das β -Galactosidase codierende Gen lacZ wurde entfernt und der Stamm mit dem für die β 1,3-N-Acetylglucosaminyltransferase aus *Neisseria meningitidis* codierenen Gen IgtA versehen um die Synthese von LNT II zu erlauben. Schließlich wurde der Stamm mit dem wbgO-Gen, das für die β 1,3-Galactosyltransferase WbgO codiert, ausgestattet. Die Gene wurden chromosomal integriert.

Das IgtA-Gen wurde hierzu zunächst in einen Expressionsvektor mit einem IPTG-induzierbaren $P_{tac}$-Promoter kloniert und dieser mit einem FRT-flankierten Chloramphenicol-Resistenzgen downstream von dem IgtA-Gen ausgestattet. Die Expressionskassette, einschließlich $P_{tac}$-Promoter, einer Ribosom-Bindungsstelle (Shine-Dalgarno-Sequenz), IgtA, FRT-cat-FRT-Resistenzmarker und einer Transkriptions-Terminator-Sequenz aus rrnB wurde mittels PCR amplifiziert. Die Kassette wurde dann chromosomal in den LacZYA-Lokus integriert.

Der Stamm wurde weiter mit einem E. coli K12 lacY-Gen unter der Kontrolle eines $P_{tac}$-Promoters versehen, um die Lactose-Aufnahme zu gewährleisten. Dazu wurde lacY in einen Expressionsvektor kloniert und anschließend durch downstream Klonierung einer FRT-kan-FRT-Resistenzkassette eine entsprechend Resistenz-markierte Expressionskassette erzeugt. Nach Amplifikation wurde diese chromosomal in den fucIK-Lokus integriert.

Für die intrazelluläre Umsetzung von LNT II zu LNT wurde das wbgO-Gen aus dem E. coli-Stamm O55:H7, das für eine β 1,3-Galactosyltransferase codiert, chromosomal in den xylAB-Lokus integriert wie für IgtA beschrieben.

Beispiel 2: Untersuchung der Bildung von LNT bzw. LNT II bei Verwendung verschiedener Kohlenstoffquellen

Trotz literaturbeschriebener Katabolitrepression (siehe McGinnis et al. J. Bacteriol. 1969, 100, 902 - 913) von Glucose auf Galactose wurde für diesen Versuch Galactose sowohl in der Mischung mit Glucose bzw. Glycerin, als auch als einzige verwertbare Kohlenstoffquelle in Minimalmedium eingesetzt, um die Produktbildung zu analysieren. Für die Versuche wurde der in Beispiel 1 hergestellte Stamm eingesetzt. Die Kulturgröße betrug jeweils 50 ml. Die Hauptkohlenstoffquellen Glucose, Glycerin und Galactose wurden jeweils mit einer Endkonzentration von 10 g $l^{-1}$ eingesetzt, während Lactose mit einer Endkonzentration von 2 g $l^{-1}$ und die beigemischte Galactose ebenfalls mit 2 g $l^{-1}$ eingesetzt wurden und zum Zeitpunkt der Induktion bei $OD_{600}$ = 0,4-0,6 (mit 0,5 mM IPTG, Endkonz.) zugegeben wurden.

Die Bildung von LNT wurde 24 Stunden nach Induktion sowohl in den Kulturüberständen, als auch in den Kulturpellets mittels HPLC Fluoreszenzphotometrisch bestimmt, nach der Derivatisierung mit Antrhanilsäure (siehe Ruhaak et al.Proteomics 2010, 10, 2330- 2336). So konnte beispielswiese eine Verbesserung der LNT-Ausbeute beim Wechsel von Glycerin zu Glucose beobachtet werden. Die Zugabe von Galactose zu der Kultur mit Glucose zeigte, wie erwartet, weder eine Auswirkung auf das Wachstum, noch auf die Produktbildung aufgrund der Katabolitrepression. Wurde neben Lactose ausschließlich Galactose als Kohlenstoffquelle verwendet oder wurde der Kultur mit Glycerin bei der Induktion Galactose zugegeben, so konnte die LNT-Konzentration in den Kulturen 24 Stunden nach Induktion deutlich gesteigert werden. Durch Zugabe von Galactose zur Kultur mit Glycerin stieg die LNT-Konzentration um den Faktor 2,7 auf

434,3 mg $l^{-1}$ und zeigte somit eine Produktbildungsrate, die etwa doppelt so hoch war wie der Wert unter Verwendung von Glucose. Wurde im Kulturmedium 10 g $l^{-1}$ Galactose ohne Glucose oder Glycerin eingesetzt, konnte eine LNT-Konzentration von 798,1 mg $l^{-1}$ erreicht werden. Somit konnte der höchste vorherige Wert, welcher mit Glucose erreicht wurde, um den Faktor 3,6 gesteigert werden (siehe Fig. 4).

Betrachtet man die Produktion des Trisaccharides LNT II, so ist zu erkennen, dass mit Glycerin als Kohlenstoffquelle vergleichsweise am meisten LNT II synthetisiert wird, während Glucose und Galactose zu ca. 16,4% weniger LNT II-Synthese führen. Werden Glucose und Galactose zusammen eingesetzt, so ist die LNT II-Konzentration 24 h nach Induktion mit nur 769 mg $l^{-1}$ deutlich geringer (siehe Fig. 5). Zu erklären ist dies möglicherweise mit der geringeren Zelldichte der Kultur. Vorstellbar ist jedoch auch ein Zusammenspiel des Induktorausschlusses durch die Glucoseaufnahme (siehe Nelson et al., EMBO J. 1983, 2, 715- 720) und der Inhibition der Lac-Permease durch die vorhandene Galactose (siehe Olsen et al., J. Biol. Chem. 1989 ,264, 15982- 15987), so dass weniger Lactose aufgenommen wird als in Systemen mit nur einer Art der Inhibition der Lac-Permease. Für diese Inhibition spricht auch, dass im Vergleich zu den Kulturen mit anderen Kohlenstoffquellen ein deutlich größeres Verhältnis von LNT zu LNT II und Lactose im Pellet vorherrscht.

Betrachtet man den LNT-Anteil im Kulturüberstand (s. Fig. 3), so wird ersichtlich, dass der Stamm auf Galactose nicht nur deutlich mehr LNT synthetisiert, sondern auch der Anteil von LNT im Kulturüberstand mit ca. 93,3% deutlich höher ist als der Anteil im Kulturüberstand der Glucose-Kulturen (ca. 54,6%). Da die Isolation von LNT aus dem Kulturüberstand im Vergleich zur Isolation aus dem Kulturpellet vorteilhaft ist, und mit Galactose als Kohlenstoffquelle auch mehr Produkt gebildet wird, ist die Synthese von LNT mit Galactose vermutlich trotz des höheren Substratpreises besonders vorteilhaft und daher bevorzugt.

Die Bildung des Tetrasaccharids LNT erfordert den Glycosyl-Transfer von N-Acetylglucosaminyl- und Galactosyl-Einheiten auf das Akzeptor-Substrat Lactose. Die jeweiligen Donor-Substrate UDP-N-Acetylglucosamin (UDP-GlcNAc) und UDP-Galactose (UDP-Gal), die für die cytosolische Glycosyltransferase-Reaktionen benötigt werden, werden - wie bereits eingangs erwähnt - durch den *E. coli*-Metabolismus bereitgestellt. Jedoch weist die unvollständige Umsetzung von Lactose zu LNT auf eine Limitierung im Angebot an Donor-Substraten, insbesondere UDP-Galactose, hin.

Um die intrazelluläre Verfügbarkeit von UDP-Glactose zu verbessern und damit eine erhöhte LNT-Ausbeute erreichen zu können, wurden Minimalmedien mit den verschiedenen Kohlenstoffquellen im Hinblick auf die Umsetzung von Lactose und die Produktbildung sowie die Freisetzung der Produkte in das Medium weiter im Detail untersucht. Dazu wurden wiederum die nach Beispiel 1 hergestellten Stämme verwendet und in Minimalmedien mit entweder 1 % Glucose, 1 % Glyzerin oder 1 % Glactose, oder mit 0.2% Galactose ergänzter 1 % Glucose bzw. 1 % Glyzerin kultiviert. Die Expression der rekombinanten Gene und die Synthese von LNT wurde in jeder Kultur durch Zugabe von IPTG (Endkonzentration 2 g $L^{-1}$) zu den Zellen in der frühen exponentiellen Wachstumsphase gestartet. 24 Stunden nach der Induktion wurden die Konzentrationen von Lactose, LNT II und LNT in jeder Kultur bestimmt.

Die Stämme wurden in LB-Medium mit 50 $\mu$g $mL^{-1}$ Chloramphenicol (um Kontaminierung zu vermeiden) bei 37 °C kultiviert. Ein Standard für Lacto-N-Tetraose wurde von IsoSep (Tullinge, Schweden) mit einer Reinheit von mehr als 95 % bezogen. Standards von UDP-Glucose-Dinatriumsalz-Hydrat (≥ 98 %) und UDP-N-Acetylglucosamin-Natriumsalz (≥ 98 %) wurden von Sigma Aldrich (Taufkirchen, Deutschland), und UDP-Galactose-Dinatriumsalz (≥ 95 %) wurde von Calbiochem (Merck, Darmstadt, Deutschland) bezogen. Lactose-Monohydrat (Ph. Eur. grade), Glucose-Monohydrat (≥ 99.5 %), Glyzerin (≥ 98 %) und Galactose (≥ 98 %) wurden von Carl Roth (Karlsruhe, Deutschland) bezogen. Alle anderen Chemikalien und Reagenzien wurden entweder von Carl Roth (Karlsruhe, Deutschland) oder Sigma Aldrich (Taufkirchen, Deutschland) mit der höchsten verfügbaren Reinheit bezogen.

Die Synthese von LNT II und LNT wurde bei 30 °C und 90 rpm in jeweils zwei Ansätzen in 500 mL Schüttelflaschen mit 50 mL Minimalmedium enthaltend 1 % der Haupt-Kohlenstoffquelle (Glyzerin, Glucose oder Galactose) und Chloramphenicol (50 $\mu$g $ml^{-1}$, um Kontamination zu vermeiden) durchgeführt. Die Zusammensetzung des Mediums war: 2.68 g $L^{-1}$ $(NH_4)_2SO_4$, 1 g $L^{-1}$ $(NH_4)_2$-H-Citrat, 10 g $L^{-1}$ Haupt-Kohlenstoffquelle (Glyzerin, Glucose oder Galactose), 14.6 g $L^{-1}$ $K_2HPO_4$, 0.241 g $L^{-1}$ $MgSO_4$, 10 mg $L^{-1}$ $MnSO_4 \cdot H_2O$, 2 g $L^{-1}$ $Na_2SO_4$, 4 g $L^{-1}$ $NaH_2PO_4 \cdot H_2O$, 0.5 g $L^{-1}$ $NH_4Cl$, 10 mg $L^{-1}$ Thiamin-Hydrochlorid, und Spurenelement-Lösung (3 mL $L^{-1}$: 0.5 g $L^{-1}$ $CaCl_2 \cdot 2H_2O$, 16.7 g $L^{-1}$ $FeCl_3 \cdot 6H_2O$, 20.1 g $L^{-1}$ $Na_2$-EDTA, 0.18 g $L^{-1}$ $ZnSO_4 \cdot 7H_2O$, 0.1 g $L^{-1}$ $MnSO_4 \cdot H_2O$, 0.16 g $L^{-1}$ Cu-$SO_4 \cdot 5H_2O$, and 0.18 g $L^{-1}$ $CoCl_2 \cdot 6\,H_2O$). Die Kulturen wurden mit einer Einzelkolonie, gewachsen auf Minimalmedium-Agarplatten mit 1 % der entsprechenden Kohlenstoffquelle, inokuliert. Nach Erreichen einer optischen Dichte bei 600 nm ($OD_{600}$) von 0.4-0.6 wurden die Kulturen mit 0.5 mM IPTG (Endkonzentration) induziert und 2 g $L^{-1}$ Lactose wurden zum Zeitpunkt der Induktion zugegeben. Um die Levels von Galactose, Lactose, LNT II und LNT zu bestimmen, wurden 2 mL Proben 24 Stunden nach der Induktion zentrifugiert (15300 g, 2 min). Nach der Zentrifugation wurden die Überstände bei -20 °C aufbewahrt bis sie derivatisiert wurden; die Pellets wurden mit 1 mL eiskalter Saline gewaschen, wie zuvor zentrifugiert, und ebenfalls bei -20 °C aufbewahrt.

Die Zell-Trockengewichte (CDW) der Kulturen mit einer Haupt-Kohlenstoffquelle (Glyzerin, Glucose oder Galactose) wurden jeweils 24 Stunden nach der Induktion durch Zentrifugation (5869 g, 4 °C, 20 min) von 10 mL Kultur

und Trocknen des Zellpellets bei 120 °C bis zum konstanten Gewicht (Minimum der zwei Ansätze) analysiert. In Schüttelflaschen wurden CDW [g $L^{-1}$] zu $OD_{600}$ [-]-Korrelationen bestimmt (0.3 für Glyzerin als Hauptkohlenstoffquelle, 0. 37 für Glucose als Hauptkohlenstoffquelle und 0.39 für Galactose als Hauptkohlenstoffquelle).

Wie in den Figuren 8a und 8b gezeigt, wird die Umsetzung von Lactose signifikant durch die zu Verfügung gestellte Kohlenstoffquelle beeinflusst und die Ergebnisse deuten darauf hin, dass die verwendeten Kohlenstoffquellen sowohl die Verschiebung zu mehr UDP-aktivierten Zuckern als auch die Lactose-Aufnahme beeinflussen. Während Kulturen, die auf Glyzerin gewachsen sind, wie oben beschrieben, zu der niedrigsten Ausbeute an LNT ($0.152 \pm 0.002$ g $L^{-1}$) führten, erhöhte die Verwendung einer Glyzerin plus Galactose-Mischung die Ausbeute an LNT wiederum um einen Faktor von fast 3. Im Gegensatz dazu zeigte der Vergleich von Kulturen gewachsen auf Glucose oder auf einer Glucose/Galactose-Mischung etwa gleiche Ausbeuten an LNT aber in Falle der Mischung war die Umsetzung von Lactose zu LNT II signifikant niedriger. Die höchste Umsetzung von Lactose, so wie auch die höchste Ausbeute an LNT ($0.810 \pm 0.013$ g $L^{-1}$) wurde in Kulturen, die auf lediglich Galactose gewachsen waren, beobachtet.

Zusätzlich zu dem Einfluss auf die Umsetzung von Lactose zu LNT II und LNT, zeigten die verwendeten Kohenstoffquellen auch einen Effekt auf die Freisetzung des Produkts LNT (siehe Fig. 8c). Während Kulturen mit Glucose oder einer Glucose/Galactose-Mischung zu einer Freisetzung von etwa 50 % des produzierten LNT führten, wurden in Kulturen mit Galactose oder Glyzerin/Galactose mehr als 90 % des gebildeten LNT im Kulturmedium gefunden.

Die Schüttelflaschen-Experimente zeigten, dass die Kohlenstoffquelle anscheinend die Bildung von LNT beeinflussen kann. Um zu bestimmen, ob die verwendeten Kohlenstoffquellen die interzelluläre Verfügbarkeit der Donor-Substrate und damit die Produktbildung steuern können, wurden die Konzentrationen von UDP-N-Acetylglucosamin, UDP-Glucose und UDP-Galactose quantifiziert. Dazu wurde der nach Beispiel 1 hergestellte Stamm in Minimalmedium mit jeweils Glyzerin, Glucose oder Galactose kultiviert, die Zellen in der späten exponentiellen Wachstumsphase geerntet und die interzellulären Metaboliten mit HPLC analysiert.

Der Stamm wurde bei 30 °C und 90 rpm in 1 L Schüttelflaschen gefüllt mit 100 mL Minimalmedium mit Glyzerin, Glucose oder Galactose, wie oben beschrieben, kultiviert. Bei einer $OD_{600}$ von 0.4-0.6, wurde die Expression mit 0.5 mM IPTG induziert und die Kulturen wurden weiter bei 30 °C und 90 rpm inkubiert. 12 Stunden nach der Induktion wurden 25 ml Proben zentrifugiert (2876 rpm, 4 °C, 15 min). Anschließend wurden die Pellets in Quenching-Puffer (Acetonitril:Methanol:$H_2O$ 4:4:2 mit 0.1 M Ameisensäure (Bennett et al. Nat. Chem. Biol., 2009, 5, 593-599)) resuspendiert und bei 4 °C kräftig auf einem Vortex alle 3 Minuten während der Inkubation für 10 min auf Eis gemischt und dann die Suspension mit 1 M $NH_4OH$ neutralisiert. Anschließend wurden die Proben wieder zentrifugiert (22410 g, 4 °C, 10 min). Die Überstände wurden in einer Speedvac CON-1000 (Fröbel, Lindau, Deutschland) getrocknet und in $H_2O$ in 5 % des Extraktionsvolumens vor der HPLC-Analyse gelöst. Die UDP-Zucker wurden mit einem Dionex-HPLC-Instrument (Thermo Fisher Scientific, Dreieich, Deutschland) ausgestattet mit Chromeleon Software, einem Gina Autosampler, P580 Pumpen, einem UVD Dioden-Array-Detektor und einer Luna C18(2) reverse Phase-Säule (250 mm x 4.5 mm, 5 $\mu$m, Phenomenex, Aschaffenburg, Deutschland) analysiert. Der folgende Gradient, modifiziert gegenüber Payne und Ames (Anal. Biochem., 1982, 123, 151-161) wurde bei einer Flußrate von 1 mL $min^{-1}$ angewandt: 0 bis 30 min linearer Gradient von 100% Lösungsmittel A/0% Lösungsmittel B zu 80% Lösungsmittel A/20% Lösungsmittel B, 30 bis 30.5 min linearer Gradient von 80% Lösungsmittel A/20% Lösungsmittel B zu 100% Lösungsmittel A/0% Lösungsmittel B, 30.5 bis 35 min isocratische Bedingungen mit 100% Lösungsmittel A zum Equilibrieren der Säule für die nächste Probe. Die Identifikation und Quantifizierung wurde bei 262 nm durch Vergleich der Retentionszeiten, Spektren und Signalflächen mit den kommerziellen Standards bei sieben verschiedenen Konzentrationen analysiert.

Das Ergebnis zeigte, dass in der Tat die Konzentration der UDP-Hexosen signifikant von der verwendeten Kohlenstoffquelle abhängt. Wachstum auf lediglich Galactose führte zu der höchsten intrazellulären Menge an UDP-Galactose ($145.63 \pm 20.52$ nmol $L^{-1}$ $OD^{-1}$), ungefähr 3 mal höher als für das Wachstum auf Glucose ($65.73 \pm 5.63$ nmol $L^{-1}$ $OD^{-1}$) oder Glyzerin ($45.87 \pm 17.42$ nmol $L^{-1}$ $OD^{-1}$) beobachtet. Die höchste Menge an UDP-N-Acetylglucosamin wurde während dem Wachstum auf Glucose beobachtet ($334.03 \pm 3.41$ nmol $L^{-1}$ $OD^{-1}$) (siehe Fig. 9).

Beispiel 3: Demonstration der Skalierbarkeit der Synthese von LNT auf Galactose

Die Verwendung von Galactose als Kohlenstoffquelle für die Ganzzell-Synthese von LNT hat im Vergleich zu den gewöhnlich verwendeten *E. coli* Kohlenstoffquellen wie Glucose oder Glyzerin einen Vorteil wegen der höheren intrazellulären UDP-Galactose-Konzentration. Zur Demonstration der Skalierbarkeit der Synthese von LNT auf Galactose wurde eine Zulauf-Kultivierung (Zulauf-Batch-Prozess) in einem Bioreaktor für hohe Zelldichten im 10-Liter Maßstab durchgeführt. Der Prozess wurde mit einem 8.45 Liter Ansatz, der eine Biomassekonzentration von etwa 13 g $L^{-1}$ CDW nach Verbrauch der anfänglich vorhandenen Galactose erreichte, gestartet. Während der folgenden Zulauf-Phase wurde die Galactose Zufuhr so eingestellt, dass eine konstante Wachstumsrate ($\mu$ = 0.054) aufrechterhalten wurde, was zu schließlich zu einer Biomasse von 55.7 g $L^{-1}$ CDW nach einer Zeit von 47 Stunden führte (siehe Fig. 10a,b).

Die Zulauf-Kultivierung des in Beispiel 1 hergestellten Stammes wurde mit Mineralsalz-Medium und Galactose als Haupt-Kohlenstoffquelle in einem 30 L Rührtank-Reaktor (Bioengineering, Wald, Schweiz) bei 30 °C mit einem

Anfangsvolumen von 8.45 L und einem Endvolumen von 13.63 L durchgeführt. Das Medium war gegenüber Wilms et al. (Biotechnol. Bioeng., 2001, 73, 95-103) modifiziert und hatte die folgende Zusammensetzung: Die 8 Liter Batch-Medium bestanden aus 2.68 g $L^{-1}$ $(NH_4)_2SO_4$, 1 g $L^{-1}$ $(NH_4)_2$-H-Citrat, 25 g $L^{-1}$ Galactose, 3.9 g $L^{-1}$ $(NH_4)_2HPO_4$, 14.6 g $L^{-1}$ $K_2HPO_4$, 0.241 g $L^{-1}$ $MgSO_4$, 10 mg $L^{-1}$ $MnSO_4$• $H_2O$, 2 g $L^{-1}$ $Na_2SO_4$, 4 g $L^{-1}$ $NaH_2PO_4$• $H_2O$, 0.5 g $L^{-1}$ $NH_4Cl$, 10 mg $L^{-1}$ Thiamin-Hydrochlorid, und Spurenelement-Lösung (3 mL $L^{-1}$, Zusammensetzung wie oben beschrieben). Während der Batch- und der Zulauf-Phase wurde der pH durch Titration mit Ammoniak (25 %) auf 7.0 reguliert. Der relative gelöste Sauerstoff ($pO_2$) wurde durch Belüftung und Bewegung mit einem Reaktor-Druck von 500 hPa über Atmosphärendruck über 40 % gehalten. Das Batch-Medium wurde zu einer Zell-Trockengewicht-Konzentration von 0.096 g $L^{-1}$ mit 0.45 L einer Über-Nacht-Vorkultur inokuliert und bei 30 °C und 90 rpm in dem Mineralsalz-Medium mit 10 g $L^{-1}$ Galactose, wie für die Schüttelflaschen oben beschrieben, kultiviert. 12.6 Stunden nach der Inokulation, bei einer Zell-Trockengewicht-Konzentration von ca. 2.4 g $L^{-1}$, wurde die Expression der rekombinanten Gene durch Zugabe von IPTG (0.5 mM Endkonzentration) induziert. Gleichzeitig wurde Lactose (16.9 g) zugegeben um die Produktbildung zu ermöglichen. Nachdem die anfänglich zur Verfügung gestellte Galactose verbraucht worden war (angezeigt durch eine Erhöhung von $pO_2$), wurde die Zulauf-Phase mit 3 Zugaben gestartet: Zugabe 1 bestand aus 514.76 g $L^{-1}$ Galactose, 15.21 g $L^{-1}$ $MgSO_4$• $7H_2O$, 0.65 g $L^{-1}$ Thiamin-Hydrochlorid und 100.89 ml $L^{-1}$ Spurenelement-Lösung (Zusammensetzung wie oben beschrieben), während Zugabe 2 aus 335.59 g $L^{-1}$ $(NH_4)_2HPO_4$ bestand und Zugabe 3 aus 150 g $L^{-1}$ Lactose für die Produktbildung bestand. Zugaben 1 und 2 wurden in einem Verhältnis von 81:19 mit einer Galactose-limitierten Wachstumsrate nach Formel (1) zugegeben

$$F(t) = \left[\left(\frac{\mu_{set}}{Y_{\frac{X}{S}}}\right) + m\right] \times \left[\frac{c_{X0} \times V_0}{c_{S0}}\right] \times e^{\mu_{set} \times t} \qquad (1)$$

mit F [L $h^{-1}$] als Zugabe-Rate, t [h] der Zeit der Zulauf-Phase, $\mu_{set}$ [$h^{-1}$] der erwünschten Wachstumsrate (in dieser Formel auf 0.1 festgesetzt), $Y_{x/s}$ [g $g^{-1}$] dem spezifischen Ausbeute-Koeffizienten der Biomasse aus dem Substrat (aus vorherigen Schüttelflaschen-Experimenten als 0.36 angenommen), m [g $g^{-1}$ $h^{-1}$] dem spezifischen Konstanhalte-Koeffizienten (als 0.04 angenommen), $c_{x0}$ [g $L^{-1}$] der Biomasse-Konzentration am Anfang der Zulauf-Phase (in diesem Prozess 12.0), $V_0$ [L] dem Kultur-Volumen am Anfang der Zulauf-Phase (auf 8.25 festgesetzt) und $c_{so}$ [g $L^{-1}$] der Galactose-Konzentration der Zugabe 1 (auf 514.76 festgesetzt) (Wenzel et al., Appl. Environ. Microbiol., 2011, 77, 6419-6425). Die Lactose-Zugabe wurde manuell angepasst basierend auf dem Verbrauch, insgesamt wurden 200.4 g Lactose zu dem System zugegeben. Das Wachstum der Zellen wurde durch Messung der $OD_{600}$ bestimmt und die Berechnung der CDW-Konzentration über den Korrelationsfaktor von 0.47 g $l^{-1}$ (bestimmt während der Fermentation) bis zu einer Kulturdichte von 40 OD-Einheiten. Danach wurden die CDW-Konzentrationen direkt zweifach durch Zetrifugation von 10 mL Kultur und anschließendem Trocknen der Zellpellets bis zu einem konstanten Gewicht in Glasröhrchen bestimmt.

Die zugegebene Lactose wurde vollständig verbraucht und während der Kultivierung zu LNT II und LNT umgesetzt. Beide, die LNT II- und LNT-Konzentrationen, stiegen während des Prozesses an und erreichten jeweils Endausbeuten von 12.72 $\pm$ 0.21 g $L^{-1}$ (LNT) und 13.70 $\pm$ 0.10 g $L^{-1}$ (LNT II). Die höchste LNT II-Konzentration wurde nach 44 Stunden (15.78 $\pm$ 0.29 g $L^{-1}$) erreicht und fiel anschließend aufgrund des Lactoseverbrauchs und der Verdünnung durch die Glactose-Zugabe ab (s. Fig. 10a,c). Um einen vollständigen Verbrauch von Lactose am Ende des Fermentationsprozesses zu gewährleisten, wurde die Lactose-Zufuhr 44 Stunden nach der Inokulation gestoppt. Die Ausbeute pro Zeit der LNT-Bildung war 0.37 g $L^{-1}$ $h^{-1}$ und die Endmenge an LNT, die im Zulauf-Batch-Prozess hergestellt wurde, war 173.37 $\pm$ 2.86 g, wobei die große Mehrheit der Produkte (88.91 $\pm$ 0.06% von LNT II und 64.86 $\pm$ 0.12% von LNT) im Überstand der Kultur gefunden wurde.

Beispiel 4: Synthese fucosylierter Oligosaccharide mit LNT- oder LNnT-Kernstruktur

Zur Synthese fucosylierter Oligosaccharide, welche LNT oder LNnT als Kernstruktur besitzen, wurde der in Beispiel 1 hergestellte Stamm zur GDP-L-Fucose-Synthese auf einem rekombinanten Weg mit den entsprechenden Fucosyltransferasen ausgestattet. Während bei der Synthese des Trisaccharids 2'-Fucosyllactose noch der *de novo* Syntheseweg von GDP-L-Fucose aufgrund der kostspieligen Fucosezugabe beim salvage Syntheseweg (siehe Baumgärtner et al., Microb. Cell Fact. 2013, 12, 40) bevorzugt wird, wird bei der Synthese größerer Oligosaccharide der salvage Syntheseweg bevorzugt. Dies ist dadurch begründet, dass bei den evtl. angestrebten Penta- und Hexasacchariden die Mehrkosten keinen starken Effekt mehr haben und, dass der salvage Syntheseweg keine GDP-L-Fucose erstellen kann ohne die Zugabe von Fucose, wodurch der Beginn der Fucosylierungsreaktionen während der Kultivierung besser gesteuert werden kann. Damit soll verhindert werden, dass bei der Verwendung gleicher Promotoren für alle rekombinant eingebrachten Gene bereits kurz nach der Induktion mit der Fucosylierung begonnen wird und infolge dessen hauptsächlich fucosylierte Lactose entsteht. Hierzu wurde das bifunktionale Enzym FKP mit L-Fucose Kinase-Aktivität und L-Fucose-1-Phosphat-Guanylyltransferase-Aktivität aus *Bacteroides fragilis* verwendet (siehe Coyne et al., Science (80-. ).

2005, 307, 1778- 1781; WO2010070104 A1). Da das Gen *fkp* schon auf einem Expressionsplasmid als funktionell bestätigt werden konnte, wurde es in den Arabinose-Degradationslokus *araBAD* des bestehenden Stammes integriert.

Zur Synthese fucosylierter LNTs wurde der Stamm mit Plasmiden mit den Genen *futC* (für die $\alpha$ 1,2-Fucosylierung, siehe Albermann et al., Carbohydr. Res. 2001, 334, 97- 103) bzw. *fucT14* (für die $\alpha$ 1,4-Fucosylierung, siehe Rabbani et al. Glycobiology, 2005, 15, 1076- 83; Rabbani et al., Biometals, 2009, 22, 1011- 7, bisher nicht beschrieben für in vivo Anwendungen in *E. coli*) oder mit dem entsprechenden Leerplasmid transformiert. Zur Synthese von fucosylierten LNnTs wurde der verwendete Stamm ebenfalls mit Plasmiden transformiert. Diese enthielten die Gene *futC* bzw. *futA* (für die $\alpha$ 1,3-Fucosylierung, siehe Ge et al. J. Biol. Chem. 1997, 272, 21357- 63). Zur Überprüfung der Produktbildung wurden die Stämme je in Minimalmedium mit Glucose (10 g $l^{-1}$) und Casamino Acids (1 g $l^{-1}$ Endkonz., Difco, für zuverlässigeres Wachstum) in Schüttelkolben kultiviert. Die Proteinexpressionen wurden dabei je bei $OD_{600}$ = 0,4-0,6 mit IPTG (0,5 mM Endkonz.) induziert und zeitgleich Lactose (2 g $l^{-1}$ Endkonz.) zugegeben. Die Zugabe von Fucose (2 g $l^{-1}$ Endkonz.) erfolgte 26 Stunden nach Induktion mit IPTG und nach vorheriger Probenahme und Zugabe von 0,5 Kulturvolumen Minimalmedium mit Glucose (10 g $l^{-1}$), um die weitere Versorgung mit ausreichend Kohlenstoff und die vorherige Synthese von ausreichenden Mengen an LNT bzw. LNnT sicher zu stellen. Anschließend wurden die Kulturen weiter inkubiert bei 30°C und 90 rpm, und es erfolgte eine zweite Probenahme 65 Stunden nach der ersten Induktion. Die Stämme zeigten im Vergleich zur Kontrolle mit Leerplasmid nach 65 Stunden jeweils Produkte, die auf die erfolgreiche Fucosylierung der Kernstrukturen zurückzuführen sind.

Beispiel 5: Synthese fucosylierter LNT- oder LNnT-Kernstrukturen mit Stämmen, bei denen die Fucosyltransferase-Gene unter die Kontrolle eines Rhamnose-induzierbaren Promoters gestellt wurden

Die gleichen Versuche wurden erneut mit Stämmen durchgeführt, die die Fucosyltransferase-Gene nicht auf Vektoren mit IPTG-induzierbarem tac-Promoter tragen, sondern in Plasmiden, welche die Fucosyltransferase-Gene (futC) unter der Kontrolle eine Rhamnose-Promoters gestellt haben (siehe Wiese, A. Molekulargenetische und funktionelle Charakterisierung des Hydantoin- Operons aus *Arthrobacter aurescens* DSM 3747. (2000)). Dabei wurde die Expression der Fucosyltransferasen-Gene erst mit L-Rhamnose (2 g $l^{-1}$) induziert als auch die L-Fucose zugegeben wurde, um mehr Proteinbildungsressourcen für die Glycosyltransferasen LgtA und LgtB/WbgO zur Verfügung zu stellen. Die sonst analog zu den vorherigen Versuchen durchgeführten Schüttekolbenkulturen zeigten mit Fucosyltransferasen stärkere Signale bei den Proben 65 h nach Induktion. Für die antibiotikafreie Synthese dieser Strukturen wurden die Gene der Fucosyltransferasen auch chromosomal in das Rhamnose-Operon *rhaBAD* integriert. Dabei stehen die integrierten Gene der Fucosyltransferasen jeweils unter einem tac-Promoter. Die Fähigkeit zur Synthese fucosylierter LNT oder LNnT konnte für alle Stämme in Schüttelkolben-Versuchen mittels HPLC und Massenspektrometrie nachgewiesen werden.

Beispiel 6: Synthese und Isolation größerer fucosylierter Oligosaccharide

Da in den zuvor genannten Versuchen auch für LNnT demonstriert werden konnte, dass die Fucosylierungen funktionieren, und da $\alpha$ 1,3- und $\alpha$ 1,2-fucosylierte Verbindungen basierend auf LNnT bereits hergestellt wurden (siehe Drouillard et al., Angew Chem Int Ed Engl 2006, 45, 1778- 1780; Dumon et al., Glycoconj. J. 2001, 18, 465- 474), sollten die hergestellten fucosylierten Verbindungen isoliert und weiter charakterisiert werden. Hierzu wurden obige Kultivierungen mit den L-Rhamnose induzierbaren Plasmiden wiederholt in 750 ml Maßstab in 3-liter Schüttelkolben mit Schikanen unter sonst gleichen Bedingungen wie zuvor. Die Produkte wurden anschließend durch Resuspension in $H_2O$, Inkubation bei 100°C für 20 Minuten und Zentrifugation aus den Zellpellets gewonnen und anschließend mittels präparativer Aktivkohle/Celite545 Chromatographie und Gelfitrationschromatographie isoliert. Dabei kam neben dem Bio-Gel P2 von Bio-Rad auch das Bio-Gel P4 (extra fine) mit engerer Partikelgrößenverteilung und größerer Ausschlussgröße zum Einsatz, da dieses für größere neutrale Oligosaccharide bereits erfolgreich zur Auftrennung verwendet wurde (siehe Priem et al., Glycobiology 2002, 12, 235- 240). Durch diese Isolationsschritte konnten aus den Kulturen insgesamt 59,4 mg LNFI (Struktur siehe Fig. 6) isoliert werden. Teile davon wurden mittels Massenspektrometrie und NMR untersucht, um die Struktur vollständig aufzuklären. Das Massenspektrum des Produktes zeigt dabei, neben wenigen Verunreinigungen, vor allem die Signale des Protonenadduktes, Natriumadduktes und Dinatriumadduktes von LNFI.

Die Oligosaccharid-Isolation aus einem mit FucT14 ausgestatteten Stamm lieferte insgesamt 133,7 mg LNDII (Struktur siehe Fig. 7). Zudem konnten 71,5 mg der fucosylierten Lacto-N-triose II isoliert werden. Die Massenspektren zeigten dabei ebenfalls die Massen der erwarteten Addukte und kaum Verunreinigungen. Es handelt sich bei den Substanzen um fucosylierte oder difucosylierte Verbindungen mit LNT als Kernstruktur, die bisher noch nicht als in vivo in *E. coli* synthetisierte Verbindungen beschrieben wurden (auch andere Synthesewege mit gleichen Produktmengen sind nicht bekannt). Eine weitere bei der Isolierung aufgetretene Verbindung ist eine Lacto-N-Pentaose mit 2 Fucosylresten, die wahrscheinlich durch die Elongation von LNT mit einer weiteren N-Acetylglucosaminylgruppe entstanden ist.

## Patentansprüche

1. Gentechnisch veränderter Mikroorganismus zur in vivo-Synthese von Lacto-N-tetraose oder Lacto-N-neotetraose in einem Mikroorganismus, wobei der Mikroorganismus

   (i) ein erstes Transgen, das für β1,3-*N*-Acetylglucosaminyltransferase codiert,
   und
   (ii) ein zweites Transgen, das für β1,3-Galactosyltransferase oder β1,4-Galactosyltransferase codiert,

   enthält, und wobei der Mikroorganismus zudem so gentechnisch verändert ist, dass die Expression von LacZ und LacA unterdrückt wird, wobei das (i) erste Transgen in den LacZYA-Lokus integriert ist und der Mikroorganismus ein weiteres Transgen enthält, das für LacY codiert.

2. Gentechnisch veränderter Mikroorganismus nach Anspruch 1, wobei das Transgen, das für LacY codiert, in den FucIK-Lokus integriert ist.

3. Gentechnisch veränderter Mikroorganismus nach einem der Ansprüche 1 oder 2, wobei der Mikroorganismus ein weiteres Transgen enthält, das für UDP-Zucker-Pyrophosphorylase codiert.

4. Gentechnisch veränderter Mikroorganismus nach einem der Ansprüche 1 bis 3, wobei der Mikroorganismus zudem so gentechnisch verändert ist, dass die Expression von UDP-Glucose-4-Epimerase unterdrückt wird.

5. Gentechnisch veränderter Mikroorganismus nach einem der Ansprüche 1 bis 4, wobei ein oder beide bzw. ein, mehrere oder sämtliche Transgene chromosomal integriert ist bzw. sind.

6. Gentechnisch veränderter Mikroorganismus nach einem der Ansprüche 1 bis 5, wobei der Mikroorganismus ein weiteres Transgen enthält, das für ein bifunktionales Enzym mit L-Fucose-Kinase-Aktivität und L-Fucose-1-Phosphat-Guanylyltransferase-Aktivität codiert sowie mindestens ein Transgen, welches für ein Enzym codiert, welches zur alpha1,2-Fucosylierung, alpha1,3-Fucosylierung oder alpha1,4-Fucosylierung befähigt ist.

7. Gentechnisch veränderter Mikroorganismus nach Anspruch 6, wobei das Transgen, das für das bifunktionale Enzym mit L-Fucose-Kinase-Aktivität und L-Fucose-1-Phosphat-Guanylyltransferase-Aktivität codiert, chromosomal integriert ist und das mindestens eine Transgen, welches für ein Enzym codiert, welches zur alpha1,2-Fucosylierung, alpha1,3-Fucosylierung oder alpha1,4-Fucosylierung befähigt ist, auf einem Plasmidvektor exprimiert wird.

8. Gentechnisch veränderter Mikroorganismus nach Anspruch 6, wobei sowohl das Transgen, das für das bifunktionale Enzym mit L-Fucose-Kinase-Aktivität und L-Fucose-1-Phosphat-Guanylyltransferase-Aktivität codiert, als auch das mindestens eine Transgen, welches für ein Enzym codiert, welches zur alpha1,2-Fucosylierung, alpha1,3-Fucosylierung oder alpha1,4-Fucosylierung befähigt ist, chromosomal integriert sind.

9. Verwendung eines gentechnisch veränderten Mikroorganismus nach einem der vorangehenden Ansprüche zur in vivo-Synthese von Lacto-N-tetraose oder Lacto-N-neotetraose oder eines fucosylierten Derivats von Lacto-N-tetraose oder Lacto-N-neotetraose in einem Mikroorganismus.

10. Verfahren zur Herstellung von Lacto-N-tetraose oder Lacto-N-neotetraose oder eines fucosylierten Derivats von Lacto-N-tetraose oder Lacto-N-neotetraose, umfassend die folgenden Schritte:

    (a) Bereitstellen eines gentechnisch veränderten Mikroorganismus nach einem der Ansprüche 1 bis 8,
    (b) Kultivieren des gentechnisch veränderten Mikroorganismus unter Bedingungen, die die Synthese von Lacto-N-tetraose bzw. Lacto-N-neotetraose erlauben,
    (c) optional Zugeben von Fucose,
    (d) optional isolieren der synthetisierten Lacto-N-tetraose bzw. Lacto-N-neotetraose bzw. des fucosylierten Derivats von Lacto-N-tetraose oder Lacto-N-neotetraose.

11. Verfahren nach Anspruch 10, wobei in Schritt (b)

    - Galactose als Kohlenstoffquelle für den Mikroorganismus verwendet wird
    oder

- Glyzerin und Galactose als Kohlenstoffquelle für den Mikroorganismus verwendet werden.

**12.** Verfahren nach einem der Ansprüche 10 bis 11, wobei in Schritt (b) kontinuierlich oder schrittweise eine oder mehrere Kohlenstoffquelle(n), vorzugsweise ausgewählt aus der Gruppe bestehend aus Lactose, Glucose, Glyzerin, Galactose und beliebige Mischungen derselben, zugegeben werden.

**13.** Verfahren nach einem der Ansprüche 10 bis 12, wobei das Verfahren als Zulauf-Batch-Prozess mit einem Batch-Volumen im Bereich von 2 bis 30 L, bevorzugt von 3 bis 20 L, besonders bevorzugt von 5 bis 15 L durchgeführt wird.

**14.** Gentechnisch veränderter Mikroorganismus nach einem der Ansprüche 1 bis 8 oder Verwendung nach Anspruch 9 oder Verfahren nach einem der Ansprüche 10 bis 13, wobei der gentechnisch veränderte Mikroorganismus ausgewählt ist aus der Gruppe bestehend aus Bakterien, Pilzen und Pflanzen, bevorzugt Mikroorganismen der Gattung *Corynebacterium, Bevibacterium, Bacillus, Saccharomyces* und *Escherichia.*

## Claims

**1.** A genetically modified microorganism for in vivo synthesis of lacto-N-tetrose or lacto-N-neotetrose in a microorganism, said microorganism comprising

(i) a first transgene coding for β1,3-*N*-acetylglucosaminyltransferase,
and
(ii) a second transgene coding for β1,3-galactosyltransferase or for β1,4-galactosyltransferase, and said microorganism in addition being genetically modified so as to suppress expression of LacZ and LacA, wherein the (i) first transgene has been integrated into the LacZYA locus and said microorganism comprises a further transgene coding for LacY.

**2.** The genetically modified microorganism according to claim 1, wherein the transgene coding for LacY has been integrated into the FucIK locus.

**3.** The genetically modified microorganism according to either of claims 1 and 2, said microorganism comprising a further transgene coding for UDP-sugar pyrophosphorylase.

**4.** The genetically modified microorganism according to any of claims 1 to 3, said microorganism in addition being genetically modified so as to suppress expression of UDP-glucose 4-epimerase.

**5.** The genetically modified microorganism according to any of claims 1 to 4, wherein one or both, or one, multiple or all, transgenes is/are chromosomally integrated.

**6.** The genetically modified microorganism according to any of claims 1 to 5, said microorganism comprising a further transgene coding for a bifunctional enzyme having L-fucokinase activity and L-fucose-1-phosphate guanylyltransferase activity, and at least one transgene coding for an enzyme capable of alpha1,2-fucosylation, alpha1,3-fucosylation or alpha1,4-fucosylation.

**7.** The genetically modified microorganism according to claim 6, wherein the transgene coding for said bifunctional enzyme having L-fucokinase activity and L-fucose-1-phosphate guanylyltransferase activity is chromosomally integrated, and the at least one transgene coding for an enzyme capable of alpha1,2-fucosylation, alpha1,3-fucosylation or alpha1,4-fucosylation is expressed on a plasmid vector.

**8.** The genetically modified microorganism according to claim 6, wherein both the transgene coding for said bifunctional enzyme having L-fucokinase activity and L-fucose-1-phosphate guanylyltransferase activity and the at least one transgene coding for an enzyme capable of alpha1,2-fucosylation, alpha1,3-fucosylation or alpha1,4-fucosylation are chromosomally integrated.

**9.** The use of a genetically modified microorganism according to any of the preceding claims for in vivo synthesis of lacto-N-tetrose or lacto-N-neotetrose or a fucosylated derivative of lacto-N-tetrose or lacto-N-neotetrose in a microorganism.

**10.** A method of preparing lacto-N-tetrose or lacto-N-neotetrose or a fucosylated derivative of lacto-N-tetrose or lacto-N-neotetrose, comprising the following steps:

(a) providing a genetically modified microorganism according to any of claims 1 to 8,
(b) culturing said genetically modified microorganism under conditions that permit synthesis of lacto-N-tetrose and lacto-N-neotetrose,
(c) optionally adding fucose,
(d) optionally isolating the synthesized lacto-N-tetrose or lacto-N-neotetrose or fucosylated derivative of lacto-N-tetrose or lacto-N-neotetrose.

**11.** The method according to claim 10, wherein step (b) comprises

- using galactose as carbon source for said microorganism
or
- using glycerol and galactose as carbon source for said microorganism.

**12.** The method according to either of claims 10 and 11, wherein step (b) comprises adding one or more carbon source(s), preferably selected from the group consisting of lactose, glucose, glycerol, galactose, and any mixtures thereof, continuously or in batches.

**13.** The method according to any of claims 10 to 12, said method being carried out by way of a fed batch process with a batch volume in the range from 2 to 30 L, preferably from 3 to 20 L, particularly preferably from 5 to 15 L.

**14.** The genetically modified microorganism according to any of claims 1 to 8 or the use according to claim 9 or the method according to any of claims 10 to 13, wherein the genetically modified microorganism is selected from the group consisting of bacteria, fungi, and plants, preferably microorganisms of the genera *Corynebacterium, Bevibacterium, Bacillus, Saccharomyces* and *Escherichia.*

## Revendications

**1.** Micro-organisme génétiquement modifié pour la synthèse in vivo du lacto-N-tétraose ou du lacto-N-néotétraose dans un micro-organisme, le micro-organisme contenant

(i) un premier transgène codant pour la β1, 3-*N*-acétylglucosaminyl-transférase,
et
(ii) un second transgène codant pour la β1,3-galactosyltransférase ou la β1,4-galactosyltransférase, et le micro-organisme étant génétiquement modifié de façon à supprimer l'expression de LacZ et de LacA, le premier transgène (i) étant intégré dans le locus LacZYA et le micro-organisme contenant un autre transgène codant pour LacY.

**2.** Micro-organisme génétiquement modifié selon la revendication 1, dans lequel le transgène codant pour LacY est intégré dans le locus fucIK.

**3.** Micro-organisme génétiquement modifié selon l'une quelconque des revendications 1 et 2, le micro-organisme contenant un autre transgène codant pour l'UDP-glucose pyrophosphorylase.

**4.** Micro-organisme génétiquement modifié selon l'une quelconque des revendications 1 à 3, le micro-organisme étant également génétiquement modifié de façon à supprimer l'expression de l'UDP-glucose-4-épimérase.

**5.** Micro-organisme génétiquement modifié selon l'une quelconque des revendications 1 à 4, dans lequel un ou les deux ou un, plusieurs ou tous les transgènes est ou sont intégré(s) au niveau chromosomique.

**6.** Micro-organisme génétiquement modifié selon l'une quelconque des revendications 1 à 5, le micro-organisme contenant un autre transgène codant pour une enzyme bifonctionnelle dotée d'une activité L-fucokinase et d'une activité L-fucose-1-phosphate-guanylyltransférase et au moins un transgène codant pour une enzyme apte à l'alpha-1,2-fucosylation, alpha-1,3-fucosylation ou alpha-1,4-fucosylation.

7. Micro-organisme génétiquement modifié selon la revendication 6, dans lequel le transgène codant pour l'enzyme bifonctionnelle dotée d'une activité L-fucokinase et d'une activité L-fucose-1-phosphate-guanylyltransférase est intégré au niveau chromosomique et l'au moins un transgène codant pour une enzyme apte à l'alpha-1,2-fucosylation, alpha-1,3-fucosylation ou alpha-1,4-fucosylation est exprimé sur un vecteur plasmidique.

8. Micro-organisme génétiquement modifié selon la revendication 6, dans lequel à la fois le transgène codant pour l'enzyme bifonctionnelle dotée d'une activité L-fucokinase et d'une activité L-fucose-1-phosphate-guanylyltransférase, et l'au moins un transgène codant pour une enzyme apte à l'alpha-1,2-fucosylation, alpha-1,3-fucosylation ou alpha-1,4-fucosylation, est intégré au niveau chromosomique.

9. Utilisation d'un micro-organisme génétiquement modifié selon l'une quelconque des revendications précédentes pour la synthèse in vivo du lacto-N-tétraose ou du lacto-N-néotétraose ou d'un dérivé fucosylé du lacto-N-tétraose ou du lacto-N-néotétraose dans un micro-organisme.

10. Procédé de préparation du lacto-N-tétraose ou du lacto-N-néotétraose ou d'un dérivé fucosylé du lacto-N-tétraose ou du lacto-N-néotétraose, comprenant les étapes suivantes consistant à :

(a) fournir un micro-organisme génétiquement modifié selon l'une quelconque des revendications 1 à 8,
(b) réaliser une culture du micro-organisme génétiquement modifié dans des conditions permettant la synthèse du lacto-N-tétraose ou du lacto-N-néotétraose,
(c) ajouter éventuellement du fucose,
(d) isoler éventuellement le lacto-N-tétraose ou le lacto-N-néotétraose synthétisé ou le dérivé fucosylé du lacto-N-tétraose ou du lacto-N-néotétraose.

11. Procédé selon la revendication 10, dans lequel, à l'étape (b)

- le galactose est utilisé comme source de carbone pour le micro-organisme
ou
- le glycérol et le galactose sont utilisés comme source de carbone pour le micro-organisme.

12. Procédé selon l'une quelconque des revendications 10 et 11, dans lequel, à l'étape (b), une ou plusieurs sources de carbone, de préférence choisies dans le groupe constitué du lactose, du glucose, du glycérol, du galactose et de mélanges quelconques de ceux-ci, sont ajoutées en continu ou étape par étape.

13. Procédé selon l'une quelconque des revendications 10 à 12, le procédé étant mis en œuvre sous la forme d'un procédé semi-continu avec un volume de lot situé dans la plage de 2 à 30 l, préférablement de 3 à 20 l, particulièrement préférablement de 5 à 15 l.

14. Micro-organisme génétiquement modifié selon l'une quelconque des revendications 1 à 8 ou utilisation selon la revendication 9 ou procédé selon l'une quelconque des revendications 10 à 13, le micro-organisme génétiquement modifié étant choisi dans le groupe constitué des bactéries, des champignons et des plantes, préférablement des micro-organismes du genre *Corynebacterium, Brevibacterium, Bacillus, Saccharomyces* et *Escherichia.*

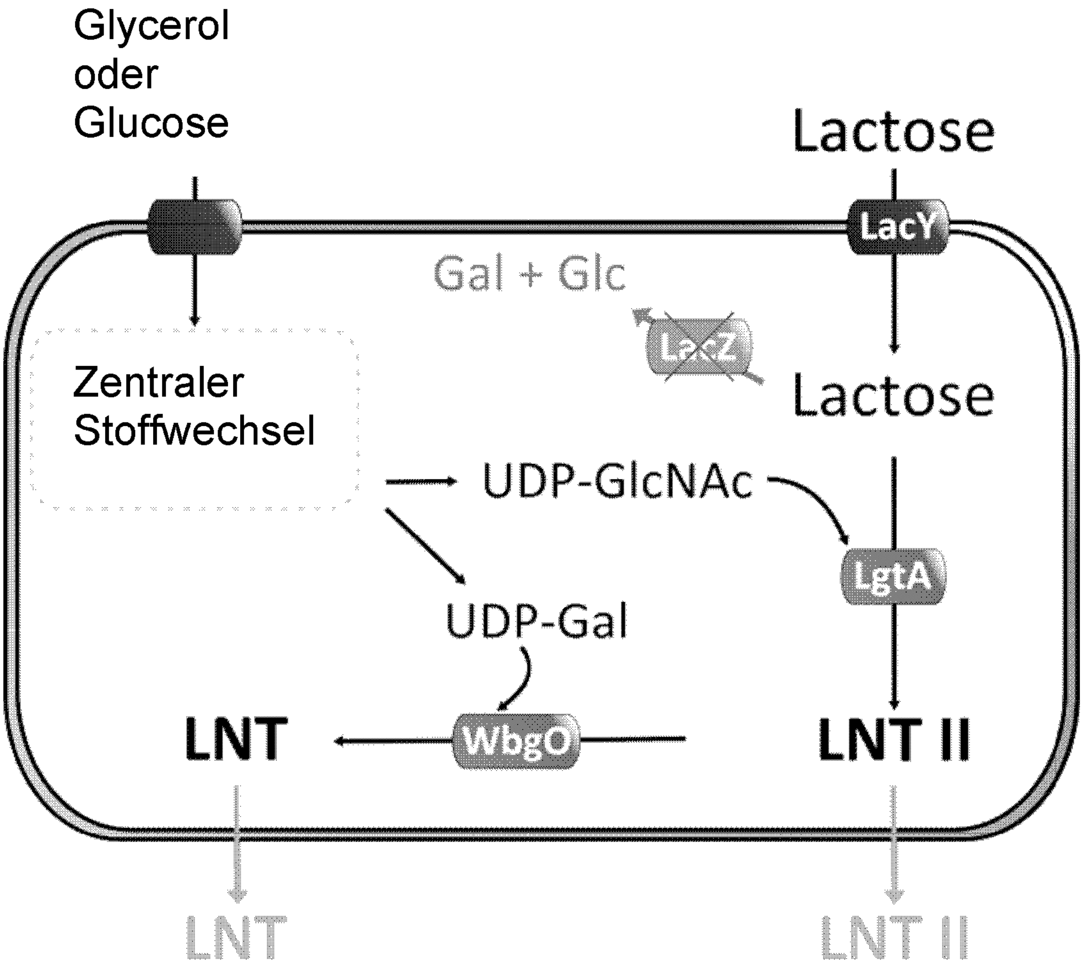
Glycerol
oder
Glucose
Lactose
LacY
Gal + Glc
LacZ
Zentraler
Stoffwechsel
Lactose
UDP-GlcNAc
LgtA
UDP-Gal
LNT
WbgO
LNT II
LNT
LNT II

Fig. 1

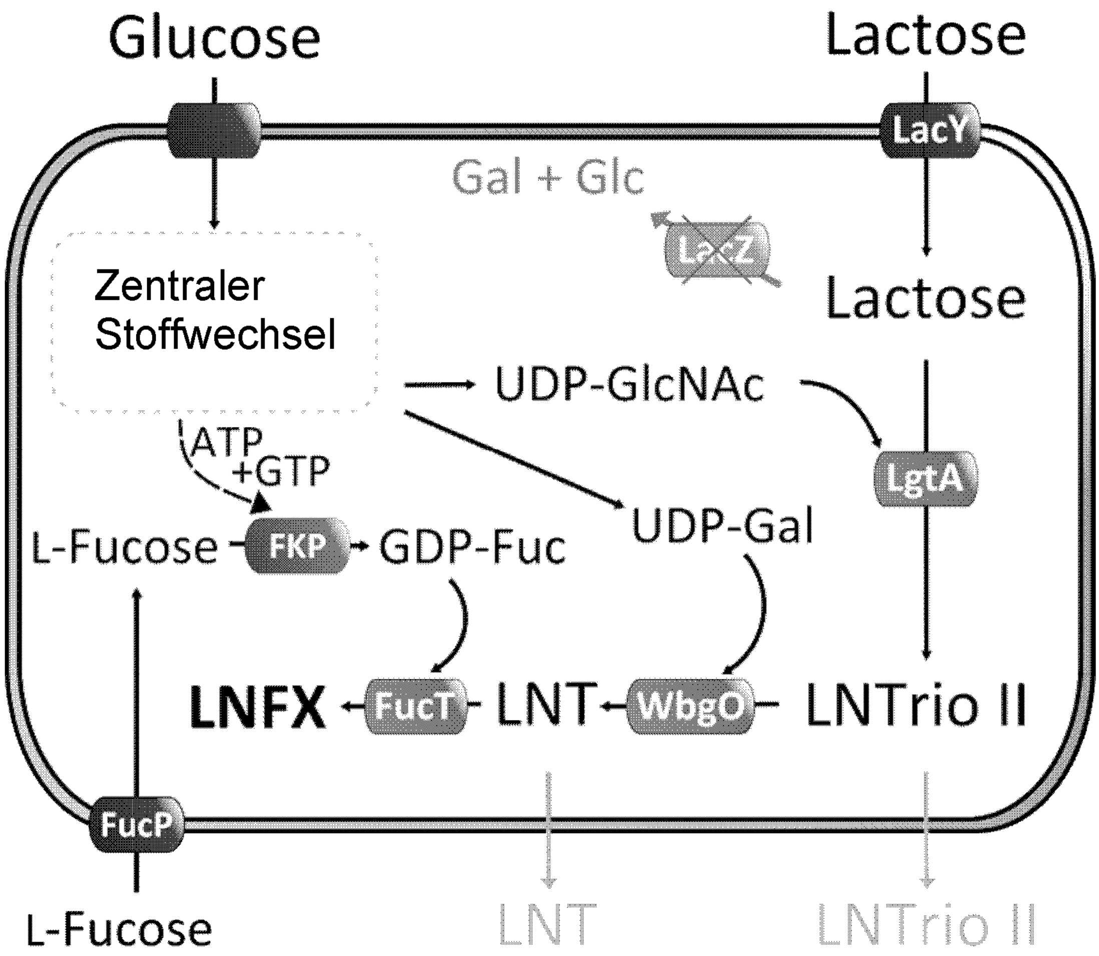
Glucose
Lactose
LacY
Gal + Glc
LacZ
Zentraler
Stoffwechsel
Lactose
UDP-GlcNAc
ATP
+GTP
LgtA
UDP-Gal
L-Fucose
FKP
GDP-Fuc
LNFX
FucT
LNT
WbgO
LNTrio II
FucP
L-Fucose
LNT
LNTrio II

Fig. 2

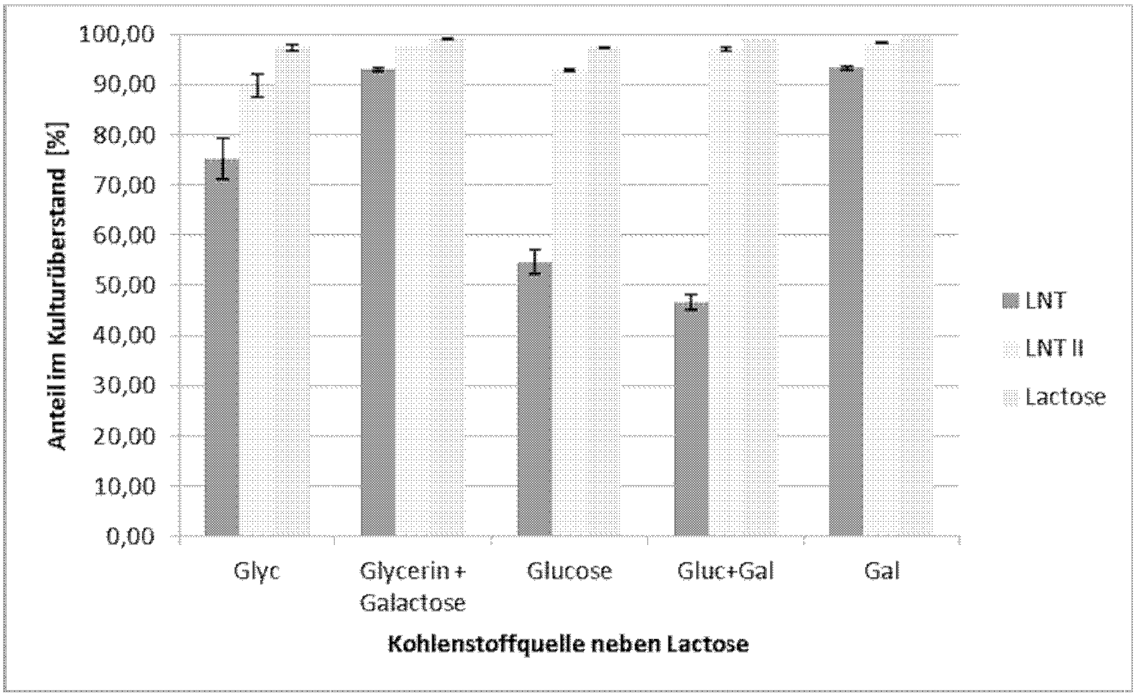
100,00
90,00
80,00
70,00
60,00
50,00
40,00
30,00
20,00
10,00
0,00
Anteil im Kulturüberstand [%]
Glyc
Glycerin + Galactose
Glucose
Gluc+Gal
Gal
Kohlenstoffquelle neben Lactose
LNT
LNT II
Lactose

Fig. 3

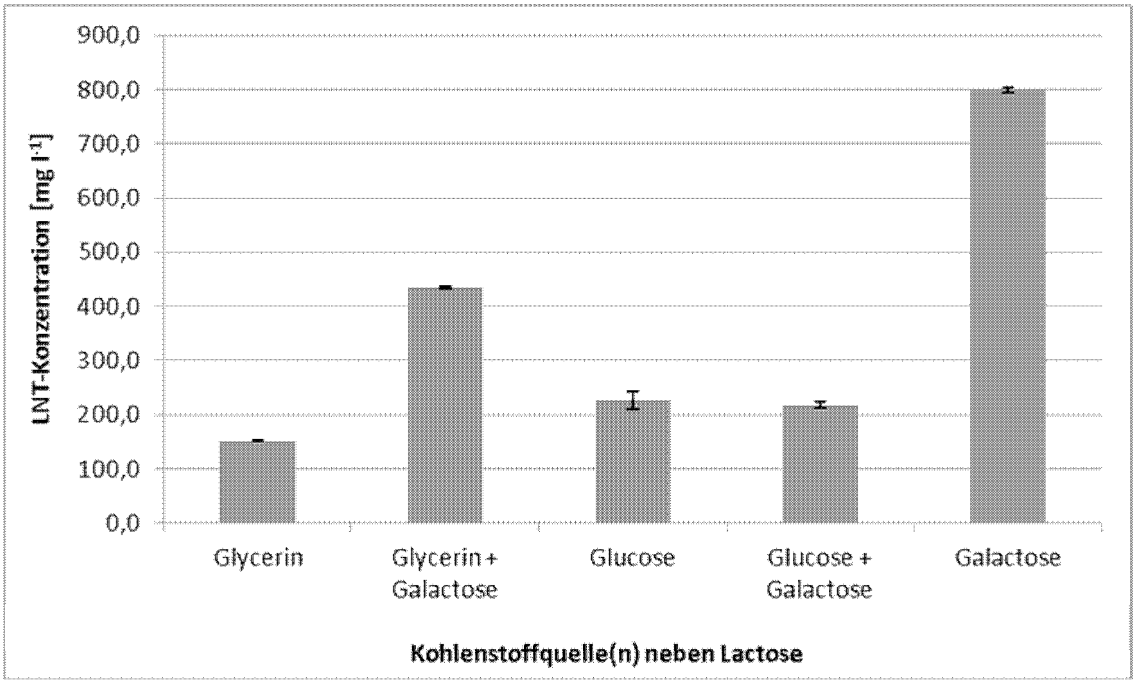
900,0
800,0
700,0
600,0
500,0
400,0
300,0
200,0
100,0
0,0
LNT-Konzentration [mg l⁻¹]
Glycerin
Glycerin + Galactose
Glucose
Glucose + Galactose
Galactose
Kohlenstoffquelle(n) neben Lactose

Fig. 4

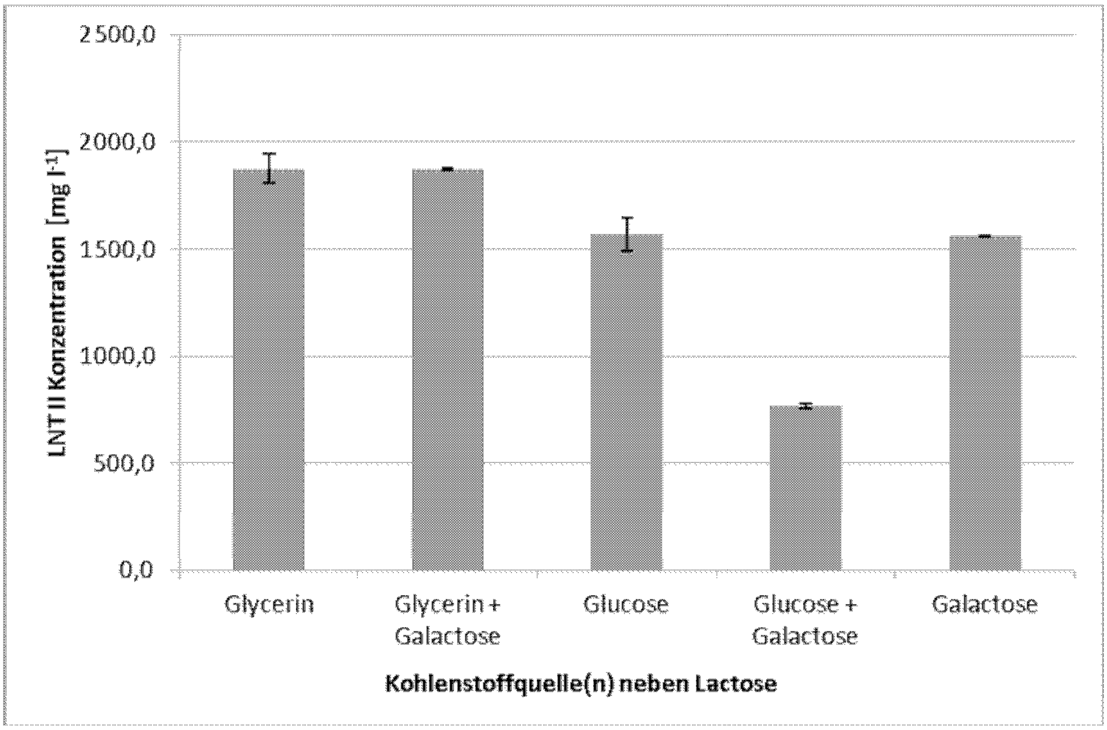
2500,0
2000,0
1500,0
1000,0
500,0
0,0
LNT II Konzentration [mg l-1]
Glycerin
Glycerin + Galactose
Glucose
Glucose + Galactose
Galactose
Kohlenstoffquelle(n) neben Lactose

Fig. 5

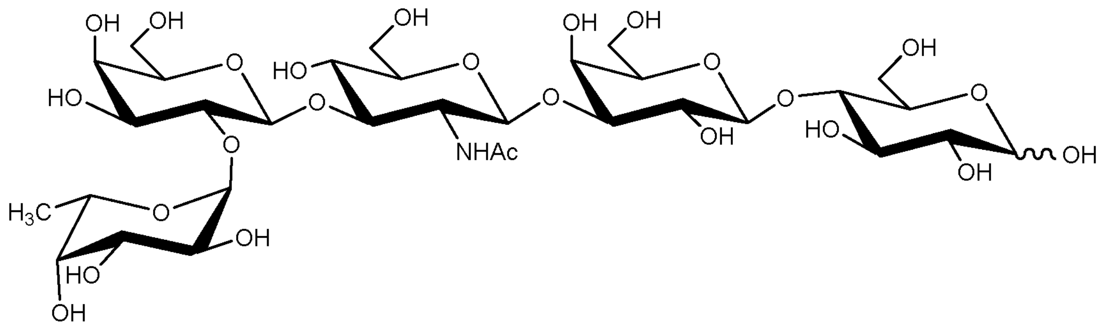

Fig. 6

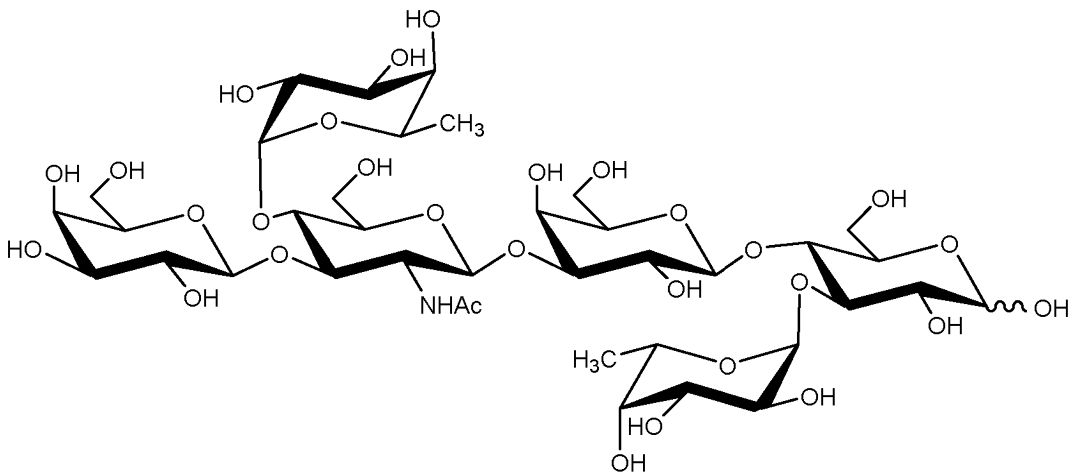

Fig. 7

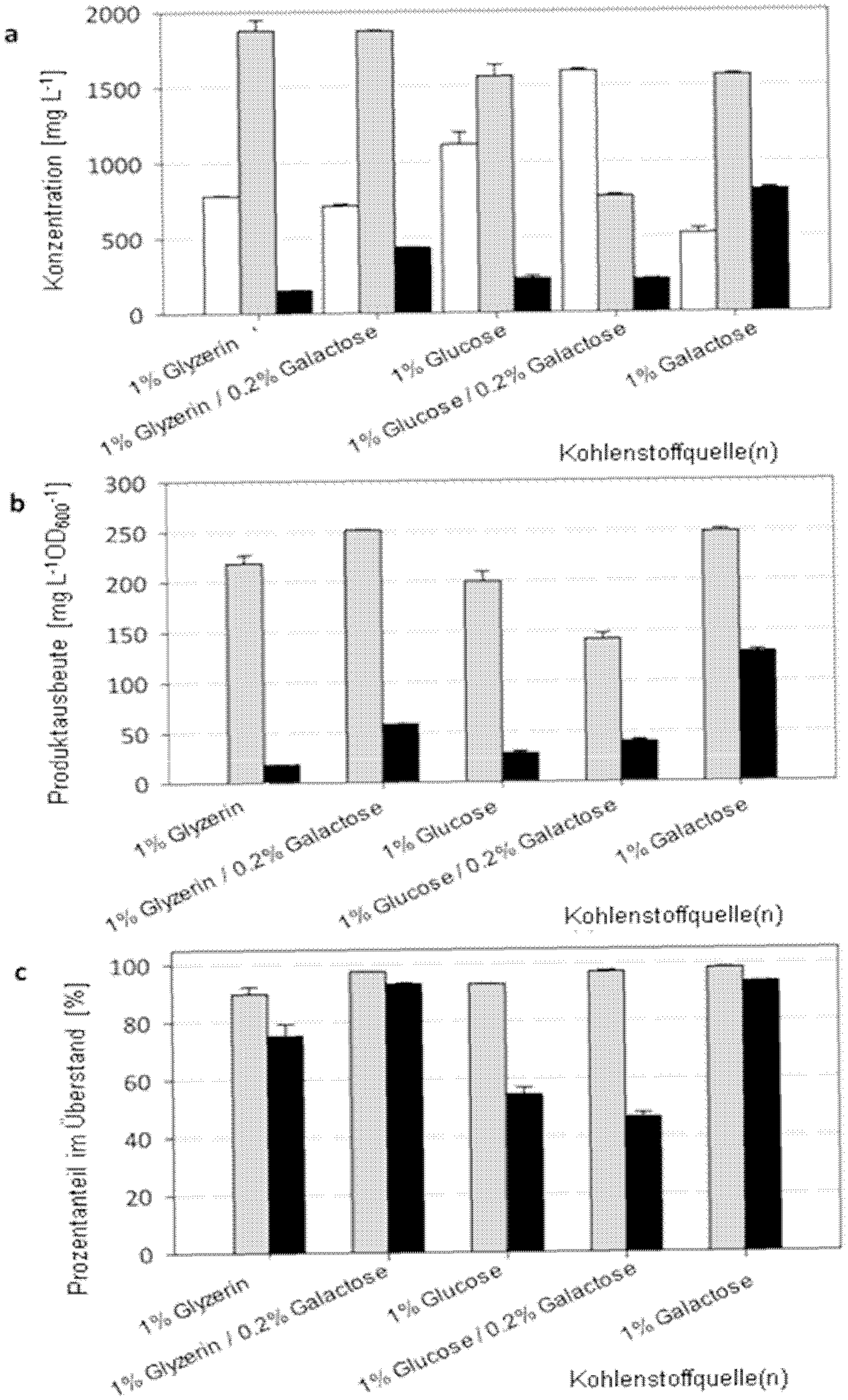
a
Konzentration [mg L-1]
2000
1500
1000
500
0
1% Glyzerin
1% Glyzerin / 0.2% Galactose
1% Glucose
1% Glucose / 0.2% Galactose
1% Galactose
Kohlenstoffquelle(n)
b
Produktausbeute [mg L-1 OD600-1]
300
250
200
150
100
50
0
1% Glyzerin
1% Glyzerin / 0.2% Galactose
1% Glucose
1% Glucose / 0.2% Galactose
1% Galactose
Kohlenstoffquelle(n)
c
Prozentanteil im Überstand [%]
100
80
60
40
20
0
1% Glyzerin
1% Glyzerin / 0.2% Galactose
1% Glucose
1% Glucose / 0.2% Galactose
1% Galactose
Kohlenstoffquelle(n)

Fig. 8

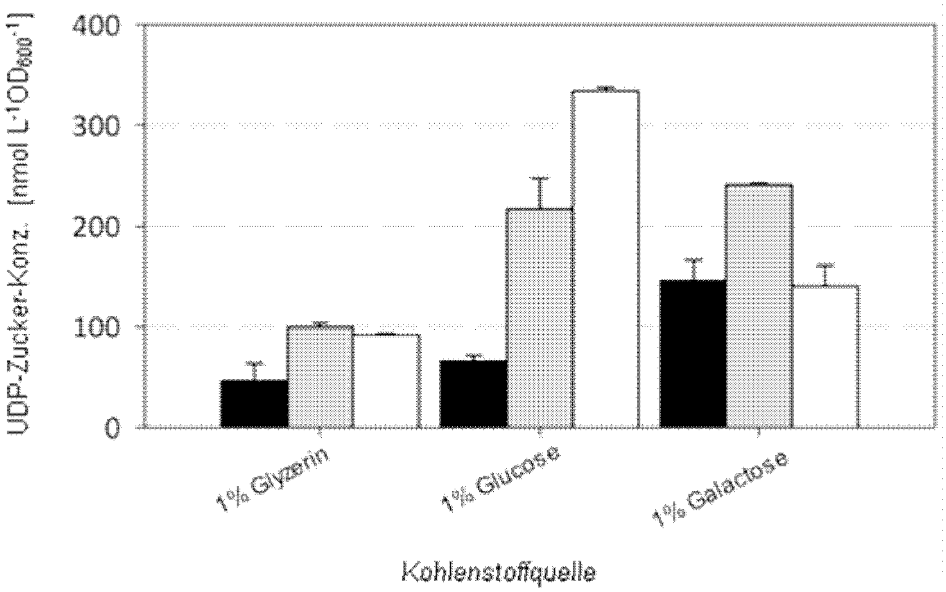
UDP-Zucker-Konz. [nmol L-1OD600-1]
400
300
200
100
0
1% Glyzerin
1% Glucose
1% Galactose
Kohlenstoffquelle

Fig. 9

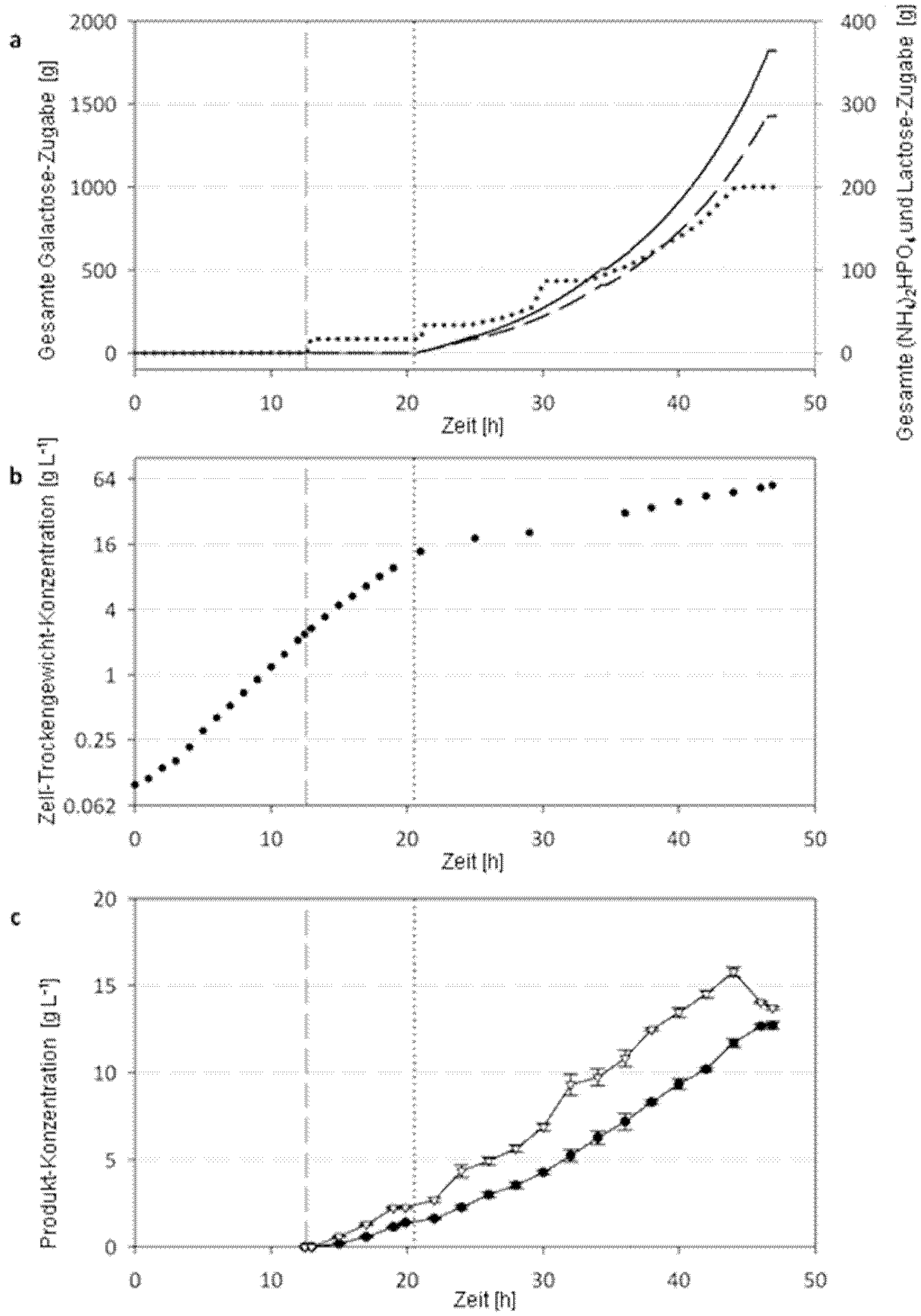
a
Gesamte Galactose-Zugabe [g]
2000
1500
1000
500
0
Gesamte $(NH_4)_2HPO_4$ und Lactose-Zugabe [g]
400
300
200
100
0
0
10
20
30
40
50
Zeit [h]
b
Zell-Trockengewicht-Konzentration [g L-1]
64
16
4
1
0.25
0.062
0
10
20
30
40
50
Zeit [h]
c
Produkt-Konzentration [g L-1]
20
15
10
5
0
0
10
20
30
40
50
Zeit [h]

Fig. 10

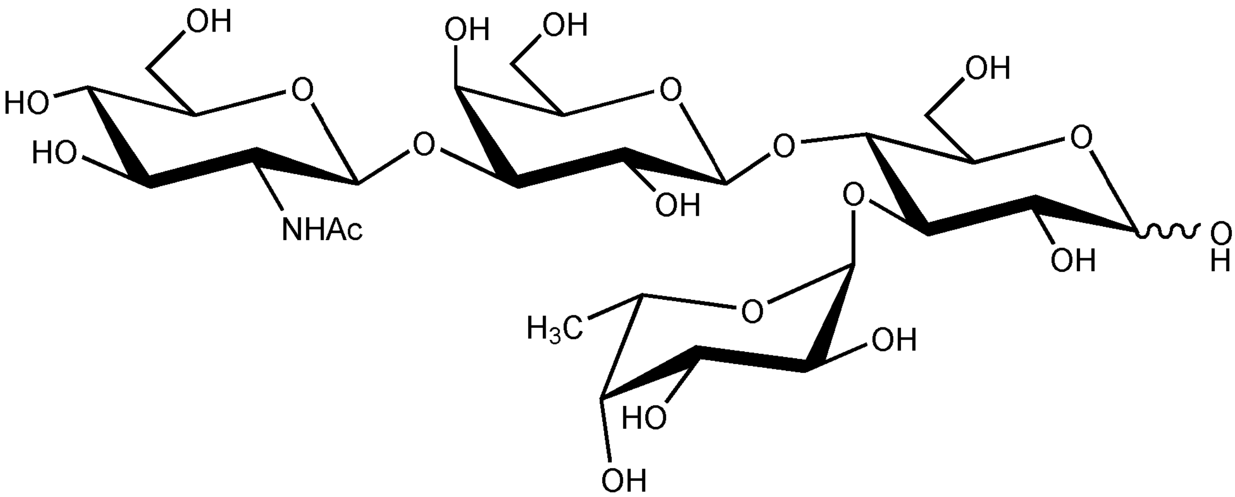

Fig. 11

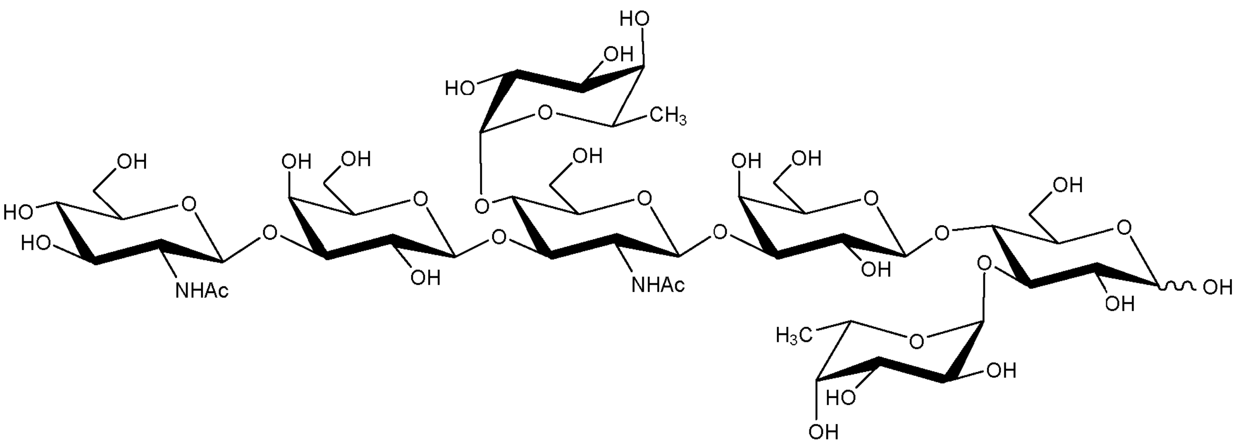

Fig. 12

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010070104 A1


### In der Beschreibung aufgeführte Nicht-Patentliteratur


- **HAN et al.** *Biotechnol. Adv.*, 2012, vol. 30, 1268-1278
- **ALY et al.** *Carbohydr. Res.*, 1999, vol. 316, 121-132
- **MURATA et al.** *Glycoconj. J.*, 1999, vol. 16, 189-195
- **FREY et al.** *FASEB J*, 1996, vol. 10, 461-70
- **RAETZ et al.** *Annu. Rev. Biochem.*, 2002, vol. 71, 635-700
- **NEIDHARDT et al.** Cellular and Molecular Biology. 1996
- **BARRETEAU et al.** *FEMS Microbiol. Rev.*, 2008, vol. 32, 168-207
- **FREY**. *FASEB J.,*, 1996, vol. 10, 461-70
- **DAMEROW et al.** *J.Biol. Chem.*, 2010, vol. 285, 878-887
- **MCGINNIS et al.** *J. Bacteriol.*, 1969, vol. 100, 902-913
- **RUHAAK et al.** *Proteomics*, 2010, vol. 10, 2330-2336
- **NELSON et al.** *EMBO J.*, 1983, vol. 2, 715-720
- **OLSEN et al.** *J. Biol. Chem.*, 1989, vol. 264, 15982-15987
- **BENNETT et al.** *Nat. Chem. Biol.*, 2009, vol. 5, 593-599
- **PAYNE** ; **AMES**. *Anal. Biochem.*, 1982, vol. 123, 151-161
- **WILMS et al.** *Biotechnol. Bioeng.*, 2001, vol. 73, 95-103
- **WENZEL et al.** *Appl. Environ. Microbiol.*, 2011, vol. 77, 6419-6425
- **BAUMGÄRTNER et al.** *Microb. Cell Fact.*, 2013, vol. 12, 40
- **COYNE et al.** *Science*, 2005, vol. 307, 1778-1781
- **ALBERMANN et al.** *Carbohydr. Res.*, 2001, vol. 334, 97-103
- **RABBANI et al.** *Glycobiology*, 2005, vol. 15, 1076-83
- **RABBANI et al.** *Biometals*, 2009, vol. 22, 1011-7
- **GE et al.** *J. Biol. Chem.*, 1997, vol. 272, 21357-63
- **DROUILLARD et al.** *Angew Chem Int Ed Engl*, 2006, vol. 45, 1778-1780
- **DUMON et al.** *Glycoconj. J.*, 2001, vol. 18, 465-474
- **PRIEM et al.** *Glycobiology*, 2002, vol. 12, 235-240